# EUROPEAN PATENT APPLICATION

(11) **EP 4 843 512 A2**
(43) Date of publication of application: **25.11.2026**
(21) Application number: 26209868.4
(22) Date of filing: 13.07.2023
(51) Int. Cl.: A61B 17/06

(54) **SUTURING DEVICES AND MECHANISMS FOR OPERATING SUCH DEVICES**

(30) Priority: 14.07.2022 US 202263389092 P
(62) Document number(s) of the earlier application(s) in accordance with Art. 76 EPC: 23839157.7 / 4 554 482
(71) Applicant: Novelrad Ltd., 0388200 Kibbutz Metzer, M.P Hefer (IL)
(72) Inventor: ROTEM, Shachar, 0388200 Kibbutz Metzer (IL); GOLDOR, Ori, 3783000 Amikam (IL); SHARABANI, Netanel, 4691500 Rishpon (IL); ROTEM, Edo, 3882000 Kibbutz Metzer (IL); SELTENREICH, Guy, 5233543 Ramat Gan (IL)
(74) Representative: Kancelaria Eupatent.pl Sp. z o.o.

(57) **Abstract**

A suturing device (10) employs a displaceable bistable mechanism to allow a user to perform successive bidirectional suture passes of a running stitch by successive presses of a single actuation button (14) as the device is rotated. The displaceable bistable mechanism may also be used in other medical and non-medical applications. Also disclosed are aspects of a suture shuttle holder, and a distal shuttle receiver, with retaining structures which generate retaining force profiles optimized for the suturing sequence.

## Description

### FIELD AND BACKGROUND OF THE INVENTION

The present invention relates to medical devices and, in particular, it concerns a suturing device. Additionally, the present invention provides various mechanisms for operating medical devices, which are suitable for operating the disclosed suturing device, but can also be used to advantage with a range of other suturing devices and other medical devices.

The various aspects of the present invention will be presented herein in the non-limiting context of a suturing device of a type similar to that described in the co-pending, co-assigned patent applications published as WO 2021/024236 A1 and WO 2021/111429 A1, which are hereby incorporated by reference as if set out herein in their entirety.

### SUMMARY OF THE INVENTION

The present invention provides mechanisms for operating medical devices, such as suturing devices, and various details of such devices.

According to the teachings of an embodiment of the present invention there is provided, a mechanism for operating a medical device comprising: (a) a handle; (b) an effector assembly mounted relative to the handle so as to be displaceable along an axial direction relative to the handle, the effector assembly comprising: (i) a first effector, (ii) a second effector, (iii) a bistable mechanism including a bistable element, and (iv) a biasing arrangement including at least a first spring element and a second spring element, the biasing arrangement biasing each of the first and second effectors in a distal direction relative to the bistable element, the bistable element assuming a first axial state in which the second effector is biased towards a first relative axial position relative to the first effector and a second axial state in which the second effector is biased towards a second relative axial position relative to the first effector; and (c) a force input deployed to selectively apply an input force to the bistable mechanism, wherein a force applied by the force input in a first direction is effective sequentially: (i) to displace the effector assembly distally along the axial direction without changing a state of the bistable mechanism, and (ii) when at least part of the effector assembly encounters an obstacle to further distal displacement, to toggle the bistable element between the first axial state and the second axial state, and wherein the biasing arrangement is configured such that the bistable mechanism can be toggled from the first state to the second state without requiring the second effector to have reached the second relative axial position.

According to a further feature of an embodiment of the present invention, the first spring element acts between the first effector and the second effector, and wherein the second spring element acts between the second effector and the bistable element.

According to a further feature of an embodiment of the present invention, the first spring element acts between the first effector and the bistable element, and wherein the second spring element acts between the second effector and the bistable element.

According to a further feature of an embodiment of the present invention, there is also provided a retraction spring deployed to return the bistable mechanism and the first and second effectors along the axial direction in a proximal direction.

According to a further feature of an embodiment of the present invention, the first effector is a holder for holding a suture needle, and wherein the second effector is an ejector effective when displaced from the first relative axial position to the second axial relative position to eject the suture needle from the holder.

According to a further feature of an embodiment of the present invention, in the second axial relative position, the ejector presents a penetrating tip.

According to a further feature of an embodiment of the present invention, at least one of the first and second springs is deployed with a preload force that defines a minimum force required to change a length of the at least one spring.

According to a further feature of an embodiment of the present invention, in the first axial state of the bistable element, the first spring is deployed with a first preload force and the second spring is deployed with a second preload force, and wherein, in the second axial state of the bistable element, at least one of the first and second preload forces changes such that a ratio between the first preload force and the second preload force differs between the first axial state and the second axial state.

There is also provided according to the teachings of an embodiment of the present invention, suturing mechanism comprising: (a) a needle having a pointed distal tip, an intermediate portion configured to receive a suture and a proximal engagement portion, the proximal engagement portion comprising a first section adjacent to the intermediate portion and a second portion proximal to the first portion, the first portion having a circumscribing cylinder of diameter D1 and length L1, and the second portion having a circumscribing cylinder of diameter D2 greater than D1 and a length L2; and (b) a holder for releasably holding the needle, the holder comprising a tube formed from super elastic material, the tube having a tip segment of length no greater than L1 having an internal diameter matching diameter D1, and a second segment having a length greater than L2 and an internal diameter matching diameter D2, such that, when the tube is forced against a proximal end of the needle, the second portion passes through the tip segment of the tube causing elastic deformation of the tip segment, and when the second portion is fully inserted within the second segment, the tube is substantially undeformed.

According to a further feature of an embodiment of the present invention, a shape of the proximal engagement portion of the needle and a design of the tube are such that a force required to extract the needle from the holder when fully inserted is greater than a force required to insert the needle into the holder.

According to a further feature of an embodiment of the present invention, a part of the tube proximal to the second segment has an internal diameter the same as the tip segment of the tube.

According to a further feature of an embodiment of the present invention, the tube continues proximally to the second segment with an internal diameter equal to the internal diameter of the second segment.

According to a further feature of an embodiment of the present invention, there is also provided an ejector element deployed within the tube and displaceable along the tube so as to eject the needle from the holder.

There is also provided according to the teachings of an embodiment of the present invention, a needle receiver for passively retaining a needle of a suturing device, the needle receiver comprising: (a) a receiver body having a needle-receiving bore extending parallel to a bore axis for receiving the needle and a retaining-element slot extending from a side of the receiver body and intersecting the needle-receiving bore; and (b) a resilient snap retainer deployed in the retaining-element slot so that the resilient snap retainer is aligned within the needle-receiving bore for resiliently retaining the needle.

According to a further feature of an embodiment of the present invention, the receiver body further comprises a locking element channel intersecting with the retaining-element slot, and wherein the resilient snap retainer is interconnected with an anchoring configuration having an aperture aligned with the locking element channel, the needle receiver further comprising a locking element deployed in the locking element channel so as to engage the aperture, thereby anchoring the resilient snap retainer in alignment with the needle-receiving bore.

According to a further feature of an embodiment of the present invention, the resilient snap retainer and the anchoring configuration are interconnected via a flexible connecting element so as to facilitate self-alignment of the resilient snap retainer with the needle inserted into the needle-receiving bore.

According to a further feature of an embodiment of the present invention, the resilient snap retainer, the flexible connecting element and the anchoring configuration are integrally formed as a unitary flat element made of super elastic material.

According to a further feature of an embodiment of the present invention, the resilient snap retainer is a snap ring.

According to a further feature of an embodiment of the present invention, the needle-receiving bore has an internally-stepped bore defining a fully-inserted position of the needle.

According to a further feature of an embodiment of the present invention, there is also provided a needle for introducing into the needle receiver, the needle having a peripheral groove for receiving the resilient snap retainer, and wherein the groove and the resilient snap retainer are configured such that a force required to release the needle from the needle receiver is greater than a force required to engage the needle in the needle receiver.

There is also provided according to the teachings of an embodiment of the present invention, a suturing mechanism comprising: (a) a shuttle having an intermediate portion configured to receive a suture and a proximal engagement portion, the proximal engagement portion presenting an axial aperture surrounded by a rim having a radius R2 from a central axis of the shuttle; (b) a shuttle receiver having a bore for receiving and releasably holding the shuttle in an inserted position, the bore having an opening which has a radius R1 and is located at an axial height H from the rim of the axial aperture of the shuttle when in the inserted position; and (c) a shuttle transmitter configuration for engaging the shuttle within the bore, the shuttle transmitter assuming a penetrating configuration in which the shuttle transmitter terminates in a penetrating point, the penetrating configuration having a gradually-increasing radius such that, at an axial distance H from the penetrating point, the penetrating configuration has a radius R3, where R3 is greater than (R1 - R2).

### BRIEF DESCRIPTION OF THE DRAWINGS

Some embodiments of the disclosure are described herein with reference to the accompanying figures. The description, together with the figures, makes apparent to a person having ordinary skill in the art how some embodiments may be practiced. The figures are for the purpose of illustrative description and no attempt is made to show structural details of an embodiment in more detail than is necessary for a fundamental understanding of the disclosure. For the sake of clarity, some objects depicted in the figures are not to scale. In the Figures:
**Figs. 1A-1C** are schematic block representations of a mechanism, constructed and operative according to an aspect of the present invention, for operating a device, such as a suturing device, the mechanism illustrated in versions employing a parallel biasing arrangement, a series biasing arrangement and a hybrid biasing arrangement, respectively;
**Figs. 2A(i)-2A(iv)** are schematic mechanical representations of a mechanism according to FIG. 1A in a first retracted state, a first extended state, a second extended state and a second retracted state, respectively;

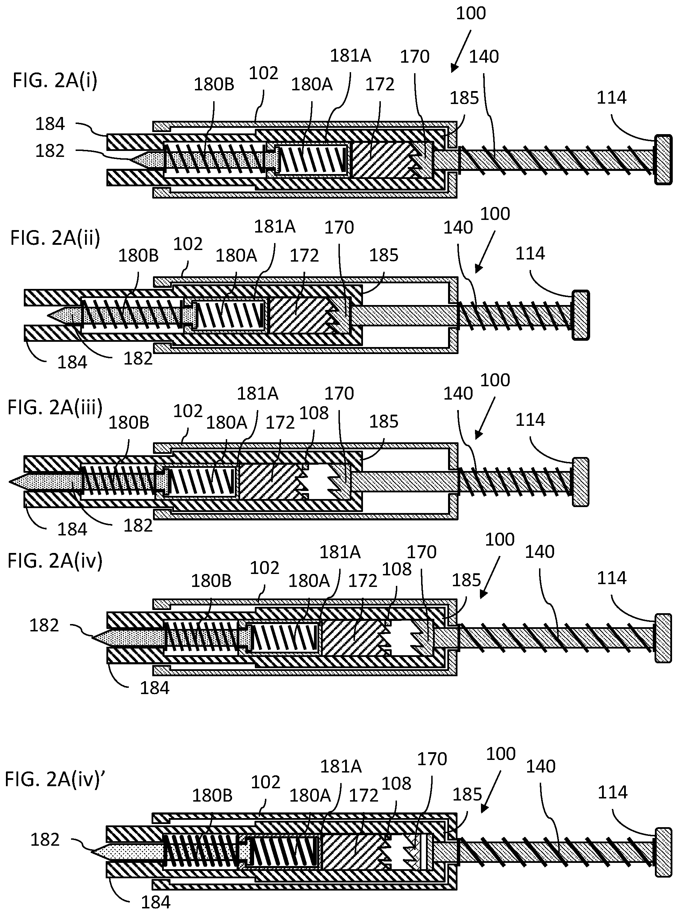

**Fig. 2A(iv)'** is a view similar to FIG. 2A(iv) illustrating a bistable mechanism with separable components;

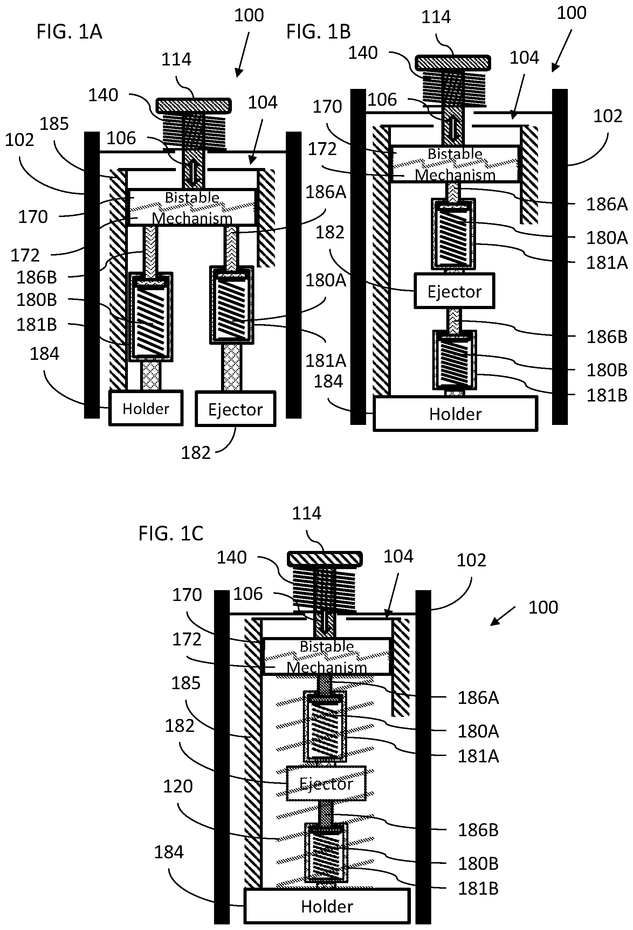

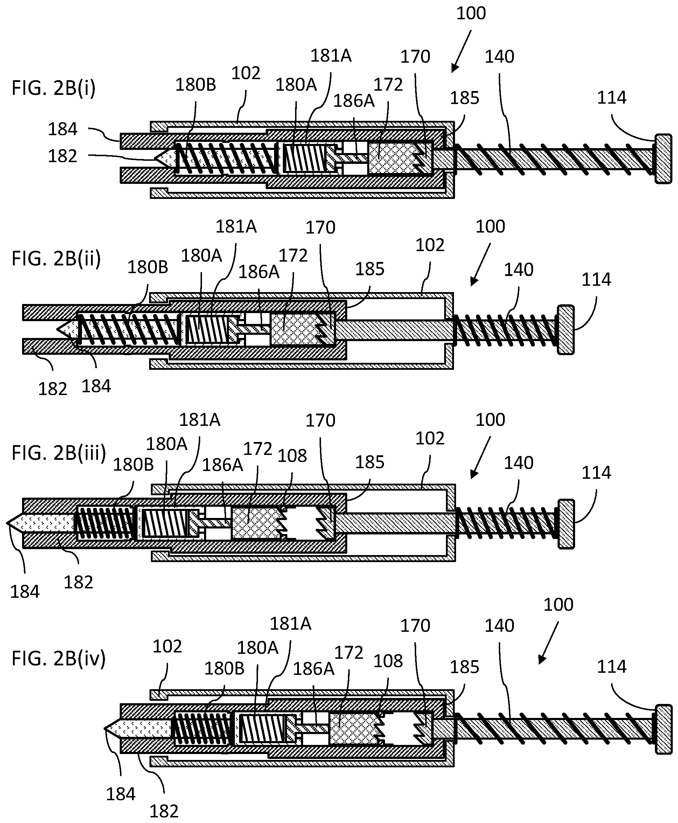

**Figs. 2B(i)-2B(iv)** are schematic mechanical representations of a mechanism according to FIG. 1B in a first retracted state, a first extended state, a second extended state and a second retracted state, respectively;

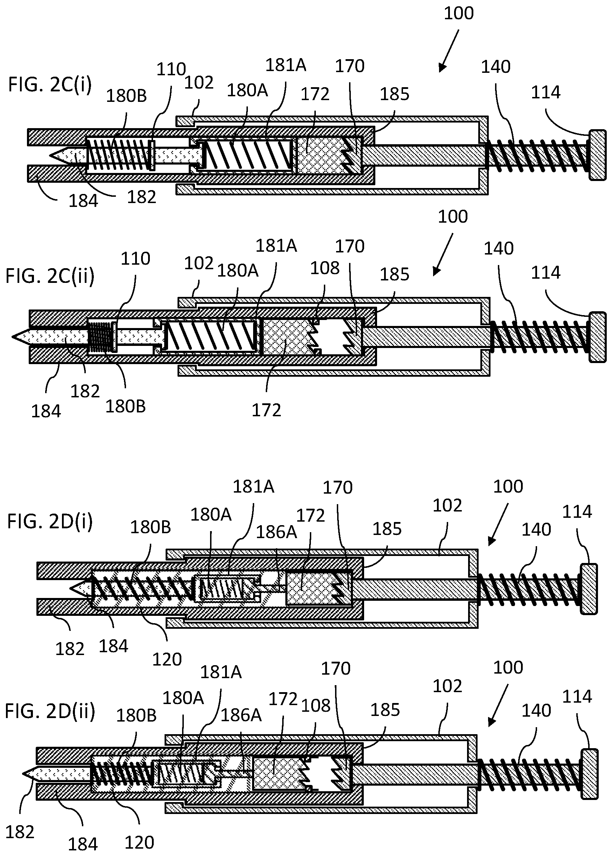

**Figs. 2C(i) and 2C(ii)** are schematic mechanical representations of an alternative mechanism according to FIG. 1B in a first extended state and a second extended state, respectively;
**Figs. 2D(i) and 2D(ii)** are schematic mechanical representations of a mechanism according to FIG. 1C in a first extended state and a second extended state, respectively;

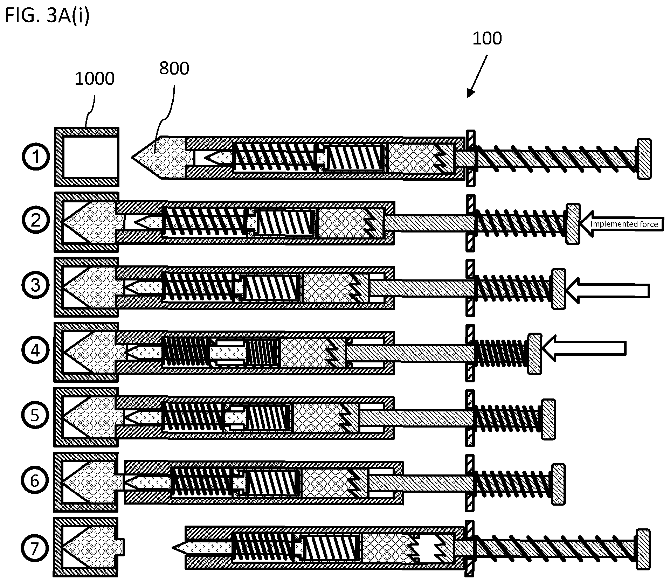

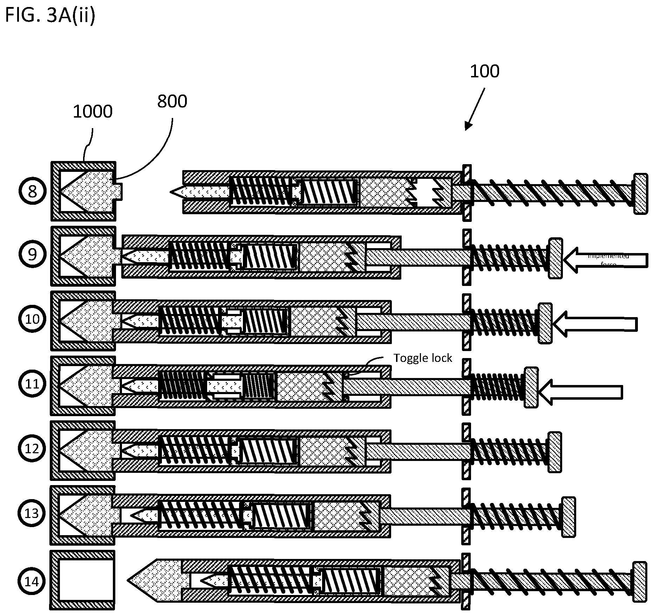

**Figs. 3A(i) and 3A(ii)** are schematic views illustrating sequential stages of operation of the mechanism of FIG. 2A employed in a suturing device for delivering a needle to a needle receiver and retrieving the needle from the needle receiver, respectively;

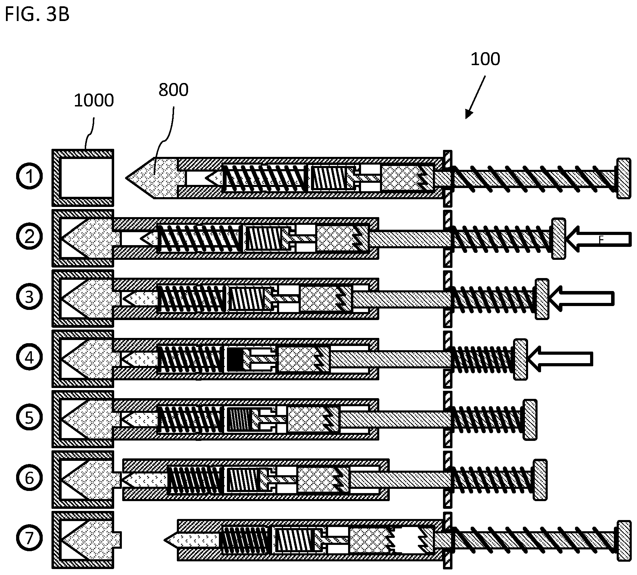

**Fig. 3B** is a schematic view illustrating sequential stages of operation of the mechanism of FIG. 2B employed in a suturing device for delivering a needle to a needle receiver;

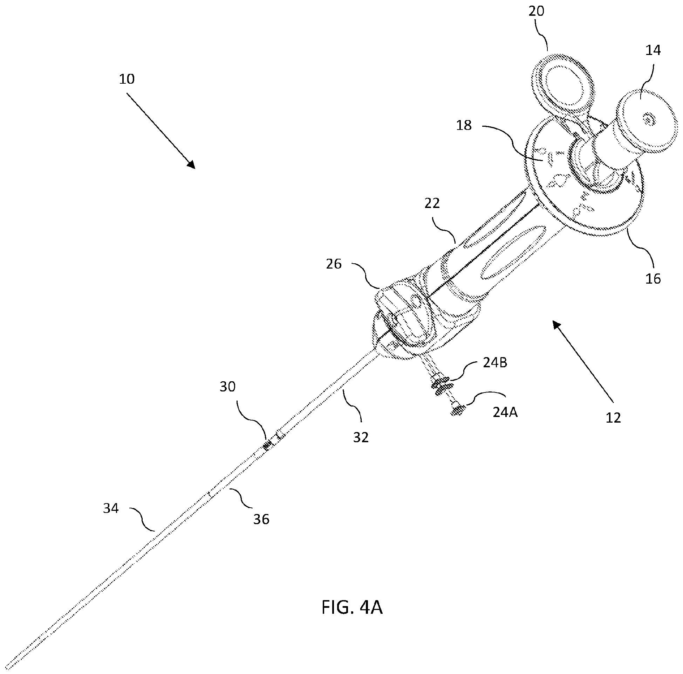

**Fig. 4A** schematically depicts a perspective view of a suturing device, according to some embodiments;

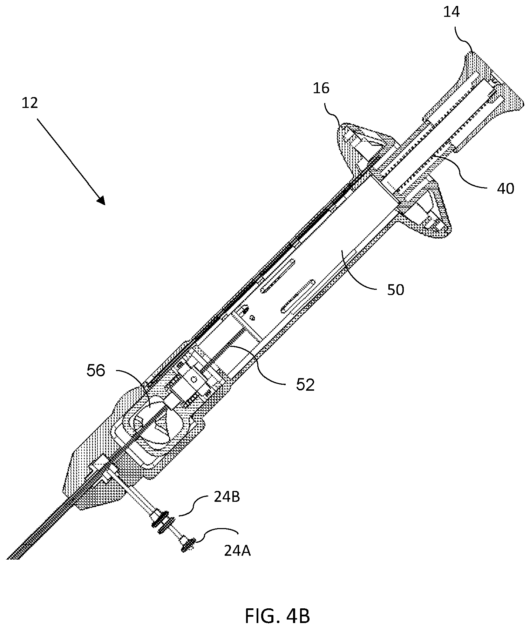

**Fig. 4B** schematically illustrates a cross-sectional view of a handle, according to some embodiments;

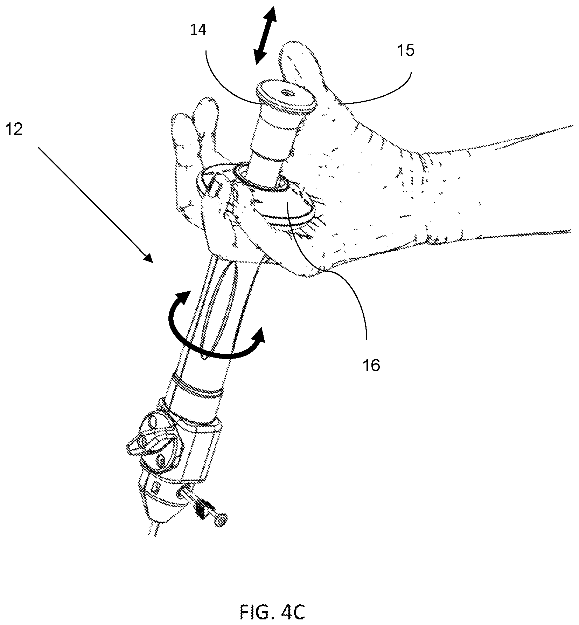

**Fig. 4C** schematically depicts a perspective view of a handle of a suturing device while being manipulated by a user; according to some embodiments;

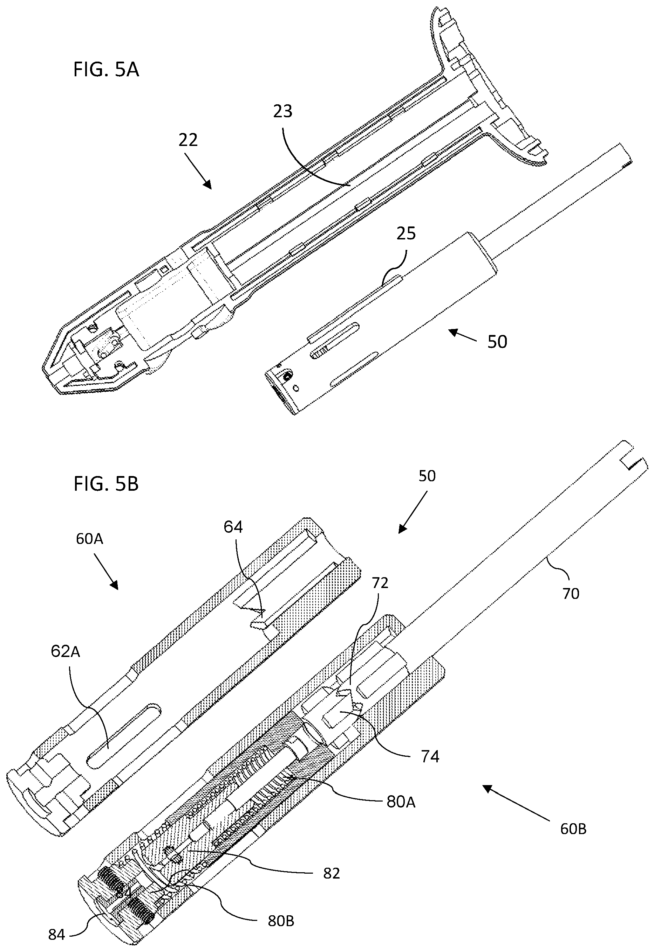

**Fig. 5A** is a partially cut-away perspective view of the inside of the handle and an exemplary click mechanism module removed from the handle;
**Fig. 5B** shows a schematic perspective cut-away view of an exemplary click mechanism module according to some embodiments;

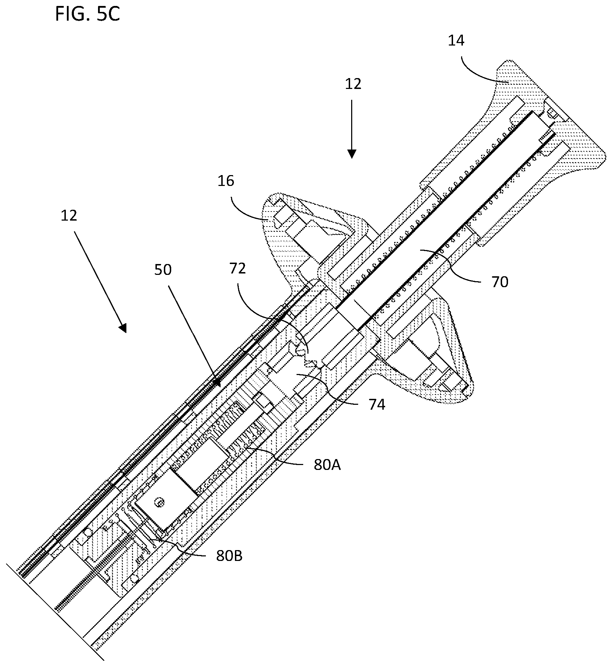

**Fig. 5C** shows a partial cross-sectional view of a handle comprising a click mechanism, according to some embodiments;

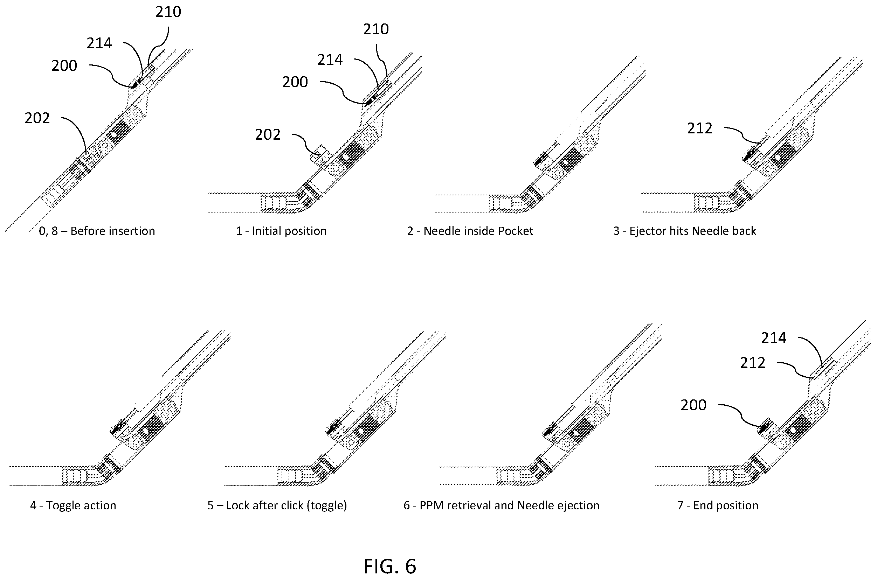

**Fig. 6** schematically illustrates in an axial cross-sectional view stages of operation of the suturing mechanism, during the performance of a suture, according to some embodiments;

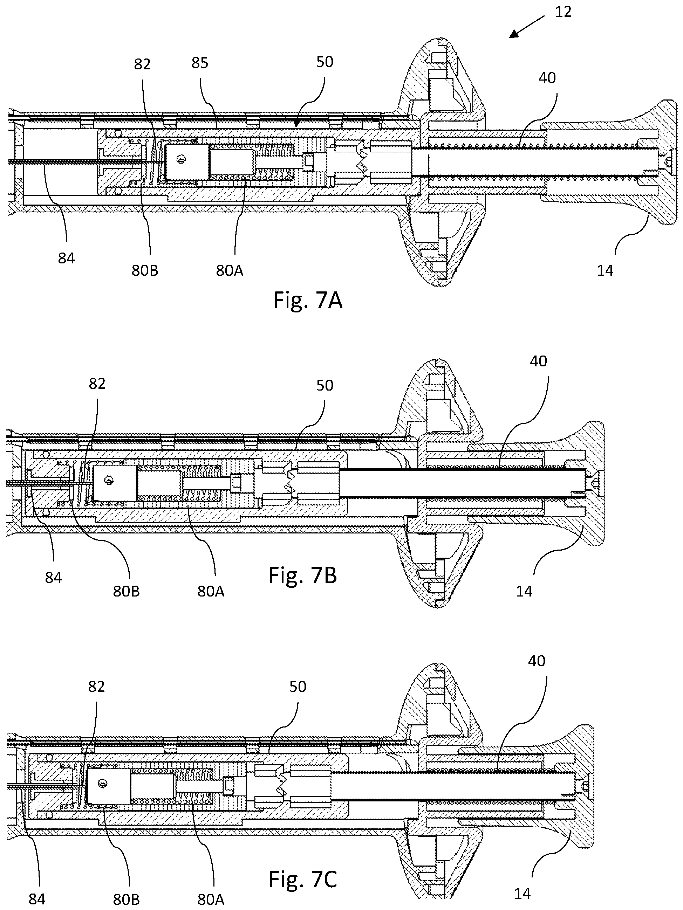

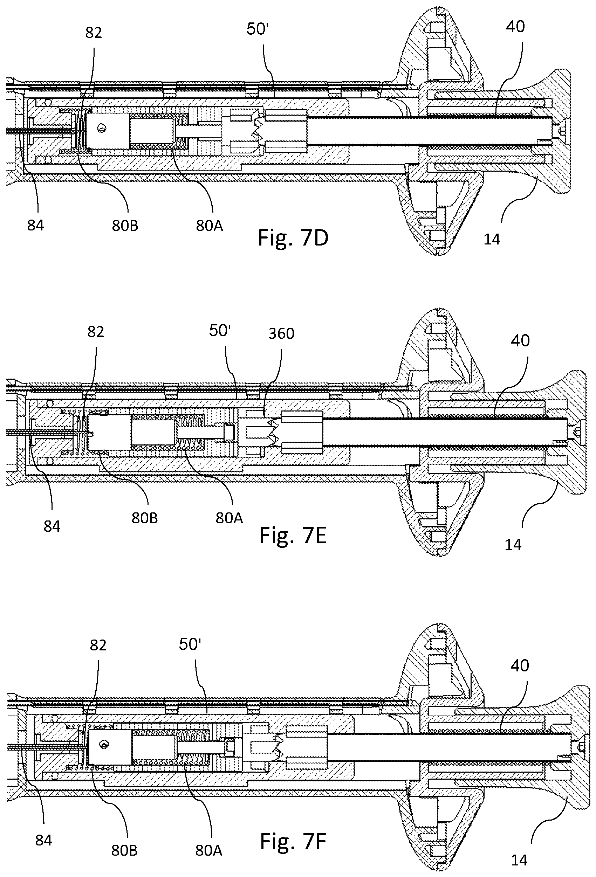

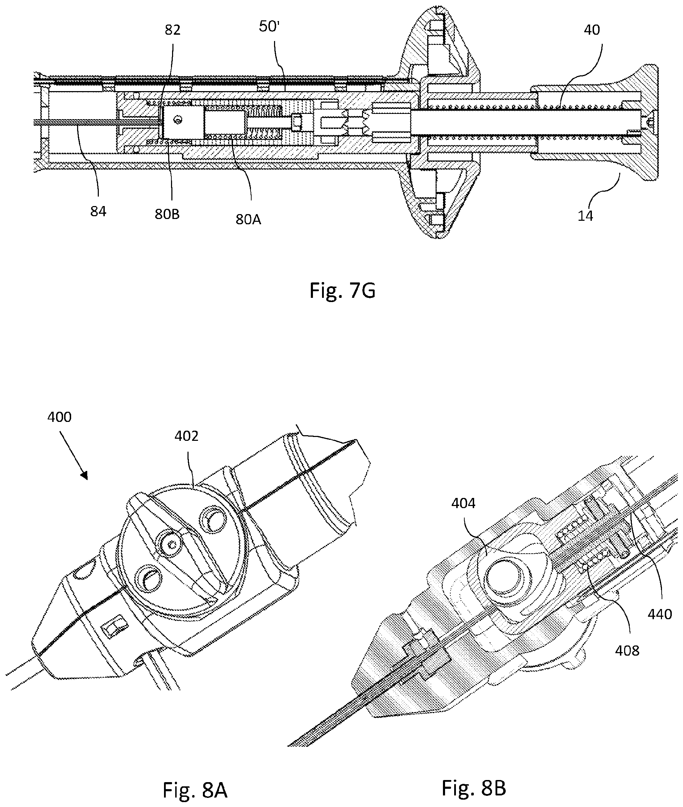

**Figs. 7A-G** show schematic cross section of a partial handle comprising a click mechanism module, at different states, according to some embodiments, where: Fig. 7A shows an initial position of the click mechanism (state 1, initial position), Fig. 7B shows state 2 (shuttle inside receiving module (pocket)); Fig. 7C shows state 3 (ejector of the shuttle transmitter module, contacts the proximal end of the shuttle); Fig. 7D shows state 4 (toggle action switching from A state to B state and vice versa); 7E shows state 5 (lock after toggle); Fig. 7F shows state 6 (shuttle transmitter module (PPM) retrieval and shuttle ejection); Fig. 7G shows end position (state 7);
**Fig. 8A** schematically shows a perspective external view of a pocket deployment module, located in the handle of the suturing device, according to some embodiments;
**Fig. 8B** shows a cut-away perspective view of the pocket deployment module of Fig. 8A,

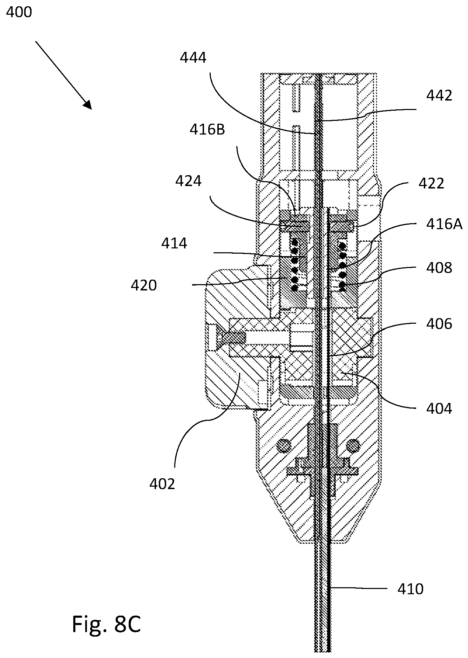

**Fig. 8C** shows a schematic cross section of a pocket deployment mechanism in a handle of a suturing mechanism, according to some embodiments;

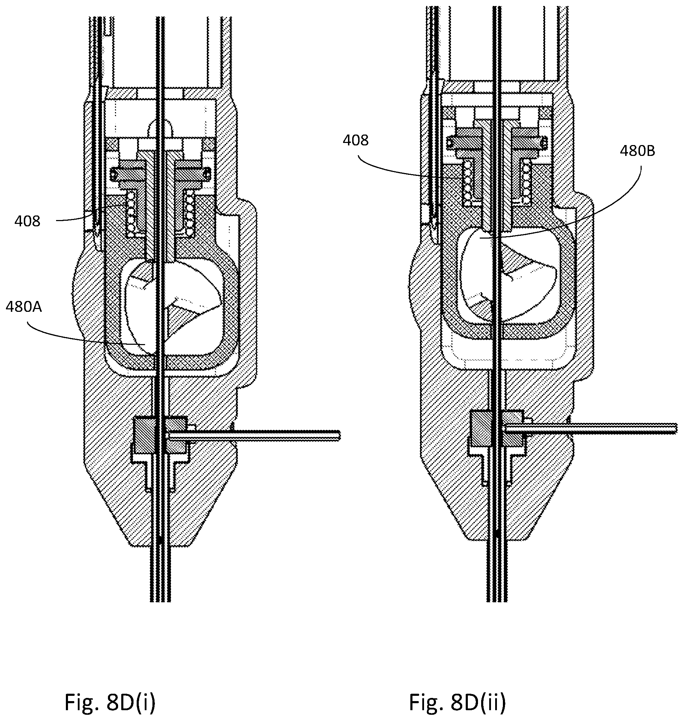

**Figs. 8D(i)-(ii)** show over the center positions for pocket retraction and pocket deployment states, according to some embodiments;

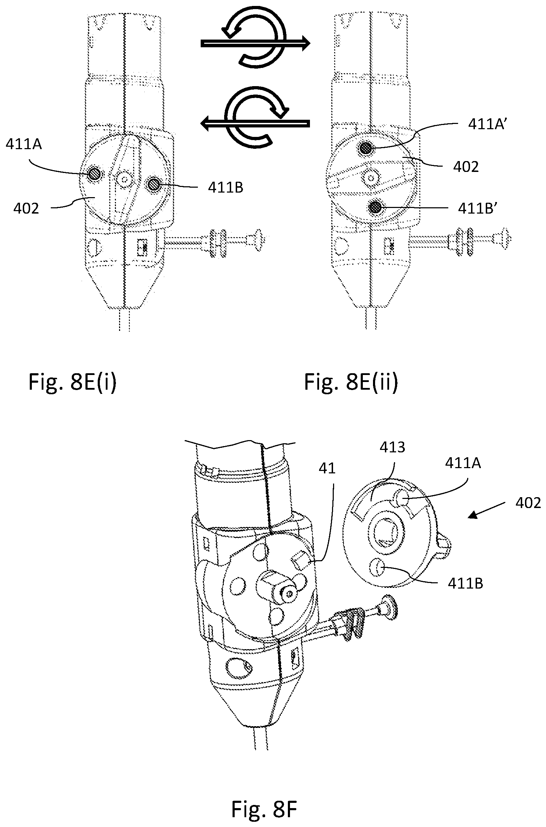

**Figs. 8E(i)-(ii)** show an exemplary visual indication with respect of the position of the pocket, in accordance with the rotation of the knob of the pocket deployment mechanism (PDM), according to some embodiments.
**Fig. 8F** shows an exemplary arrangement for defining the end of cam rotation movements of the PDM, according to some embodiments;

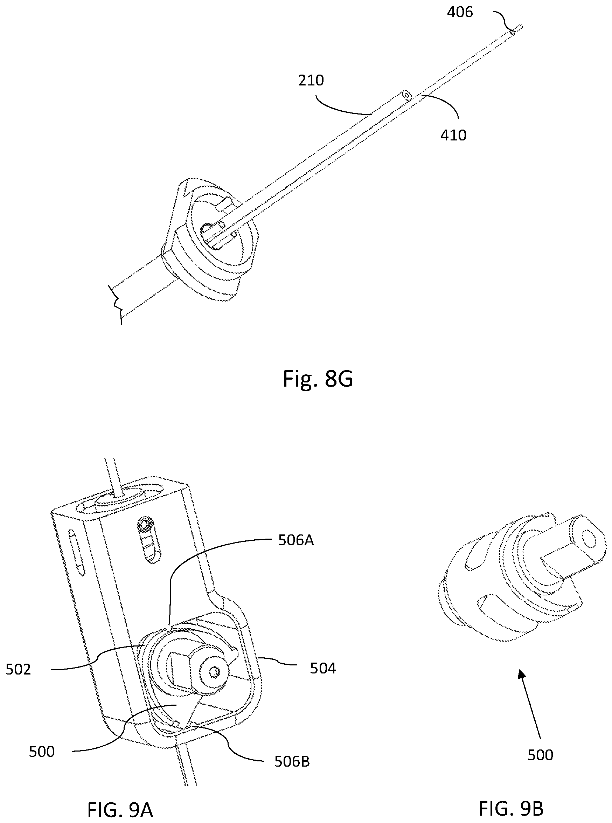

**Fig. 8G** shows a schematic illustration of a connection element of a pocket deployment mechanism and a corresponding supporting tube, according to some embodiments.
**Fig. 9A** shows an illustration of an exemplary cam and follower module, allowing tactile feedback, according to some embodiments;
**Fig. 9B** is a perspective view of a cam from Fig. 9A;

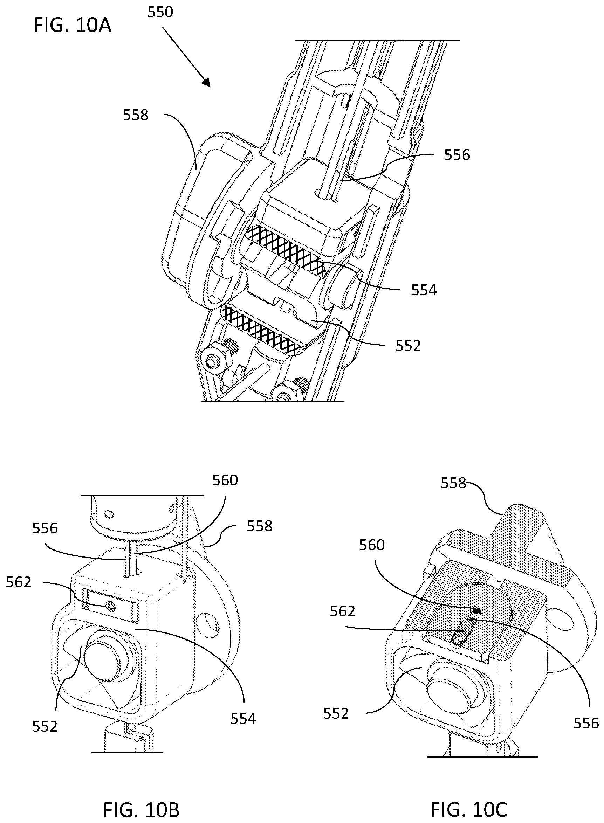

**Figs. 10A-C** are partially cut-away perspective views of an exemplary pocket deployment module, according to some embodiments.

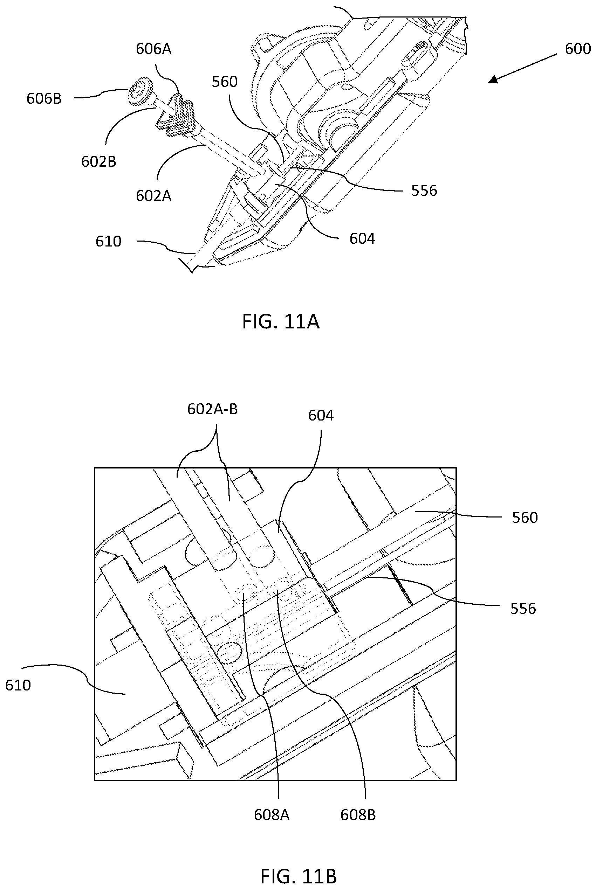

**Fig. 11A** is a cut-away perspective view of a bleeder assembly according to some embodiments showing external bleeder tubes extending from a handle of suturing device, which are connected/routed via a manifold to internal bleeder tubes;
**Fig. 11B** is an enlarged schematic representation of flow paths within a manifold from the bleeder assembly of Fig. 11A;

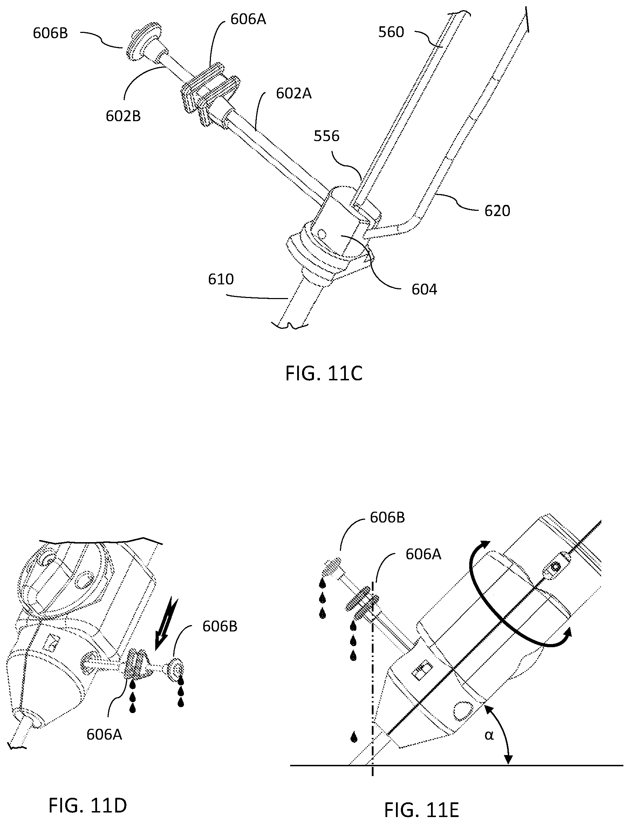

**Fig. 11C** is a perspective view of a manifold of the bleeder assembly of Fig. 11A;
**Fig. 11D and Fig. 11E** show enlarged perspective views of bleeder drip stoppers and illustration of blood drops dripping therefrom;

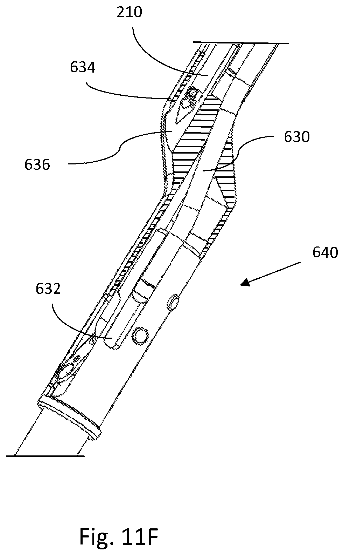

**Fig. 11F** is a partially cut-away enlarged view of the suturing mechanism region of a suturing device, illustrating distal components of the bleeders assembly, including internal bleeder tubes and their respective bleeder openings;

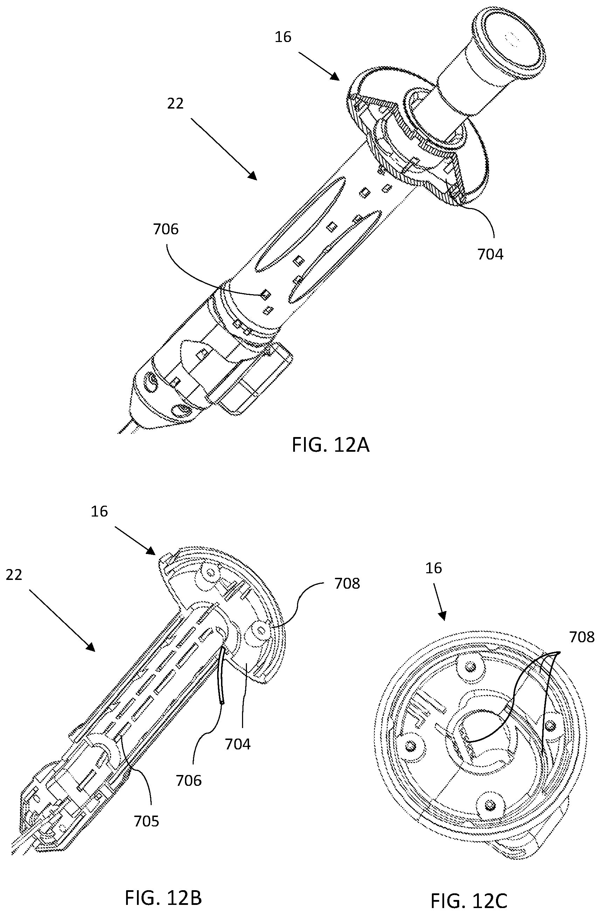

**Figs. 12A-C** are partially cut-away views showing routing of a suture thread tube through a handle of a suturing device, according to some embodiments, showing a perspective view with a proximal flat torus element cut away, a perspective view of the inside of a handle casing, and a top view of a cut-away flat torus element, respectively;

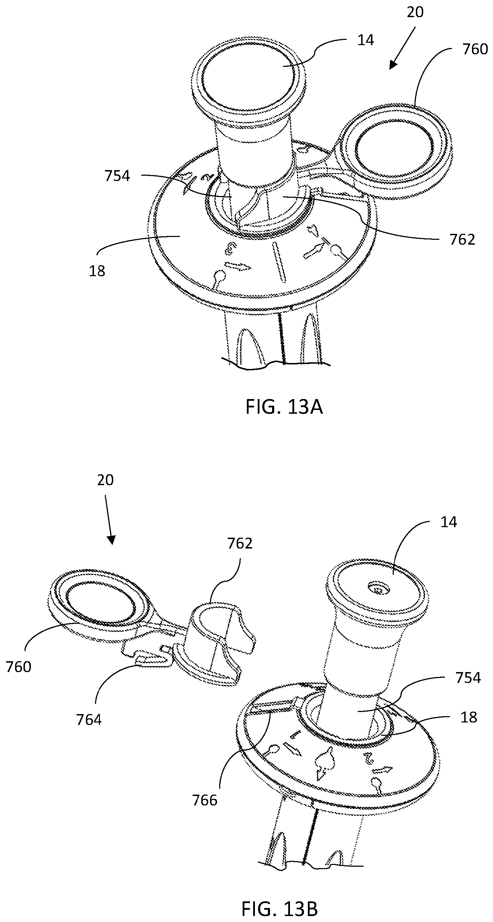

**Figs. 13A-13B** are isometric views illustrating a safety catch mechanism, according to some embodiments, in an engaged and a disengaged state, respectively;

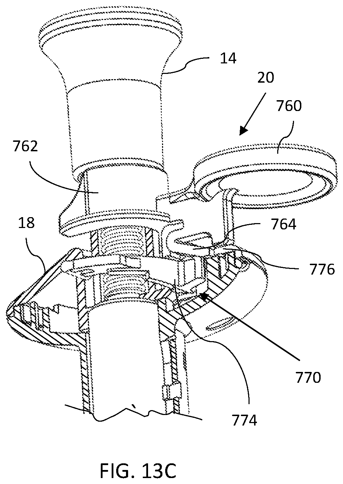

**Fig. 13C** shows a partially cut-away view of a flat torus element, showing internal portions of an internal safety catch engagement mechanism;

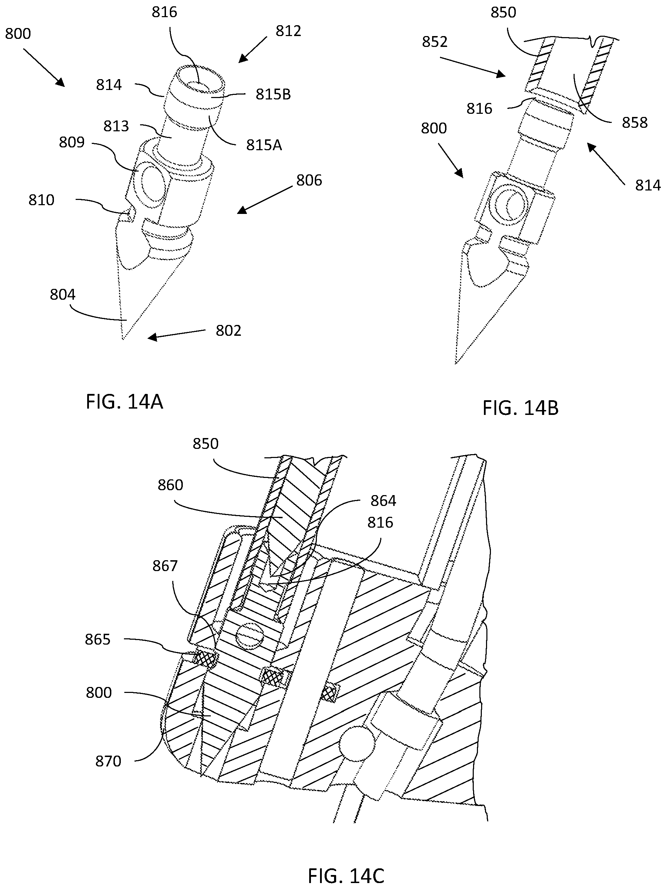

**Fig. 14A** shows a perspective view of an exemplary shuttle, according to some embodiments;
**Fig. 14B** is a partially cut-away perspective view of a shuttle and a proximal region of a holder (tube);
**Fig. 14C** is a partially cut-away perspective view of a shuttle-shuttle transmitter module interface, while being associated with a shuttle receiver module;

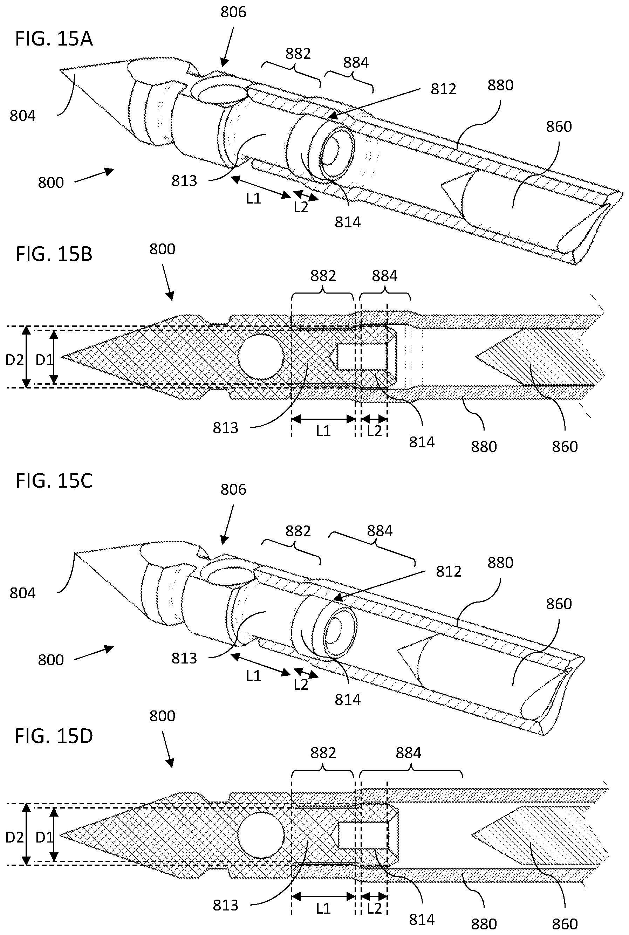

**Fig. 15A** is a partially cut-away perspective view showing engagement of a shuttle needle with a shuttle holder tube according to a further aspect of the present invention;
**Fig. 15B** is an axial cross-sectional view of the arrangement of Fig. 15A;
**Figs. 15C and 15D** are views similar to Figs. 15A and 15B, respectively, for a variant implementation of the shuttle holder tube;

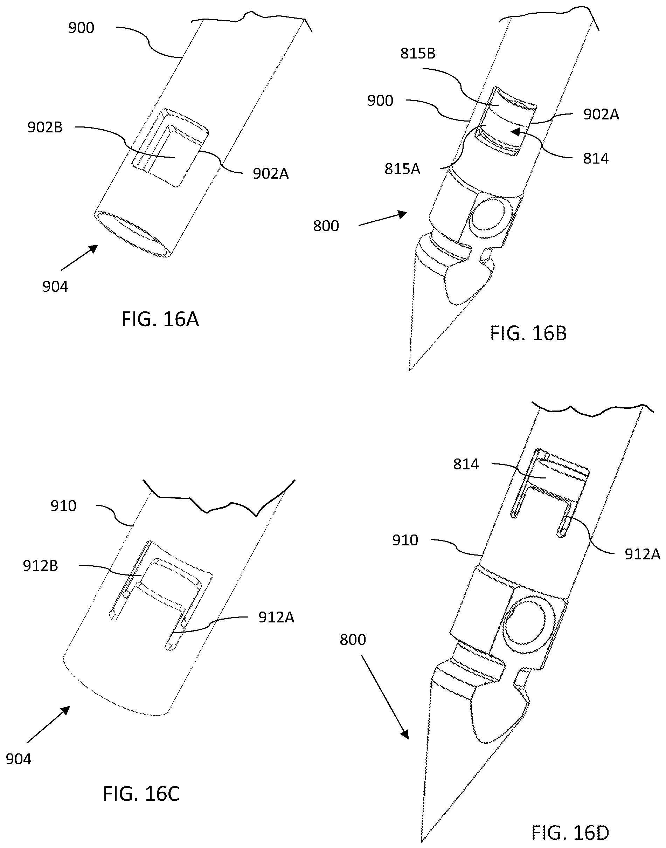

**Fig. 16A** is a perspective view of an exemplary configuration of a distal region of a shuttle holder (PPM) tube for interfacing with a shuttle, according to some embodiments, having two enlarged openings;
**Fig. 16B** is a perspective view of the shuttle holder tube of Fig. 16A associated with a shuttle;
**Figs. 16C and 16D** are views similar to Figs. 16A and 16B, respectively, illustrating a distal end of a PPM tube, having two partial openings;

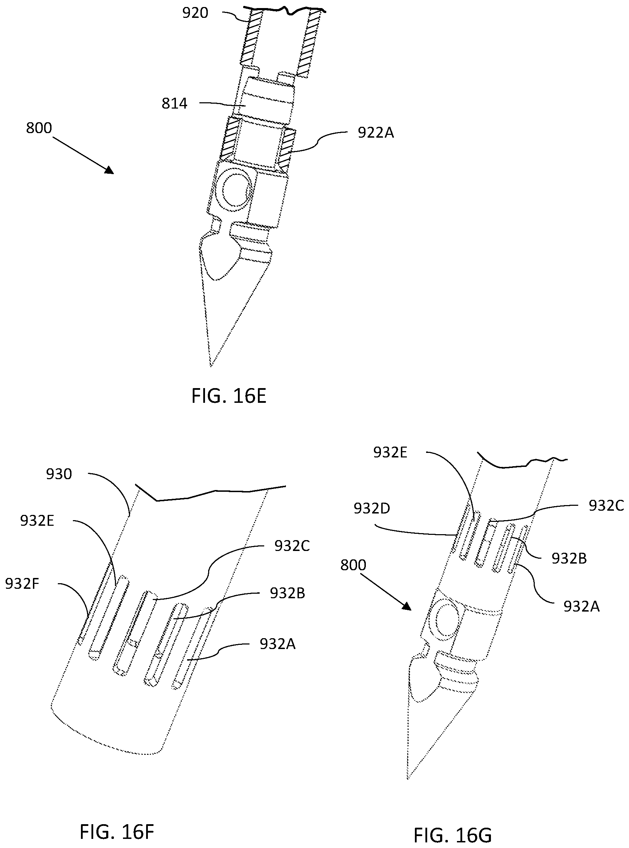

**Fig. 16E** is a partially cut-away perspective view showing a distal end of a PPM tube, having openings;
**Figs. 16F and 16G** are views similar to Figs. 16A and 16B, respectively, illustrating a distal end of a PPM tube, having slotted openings;

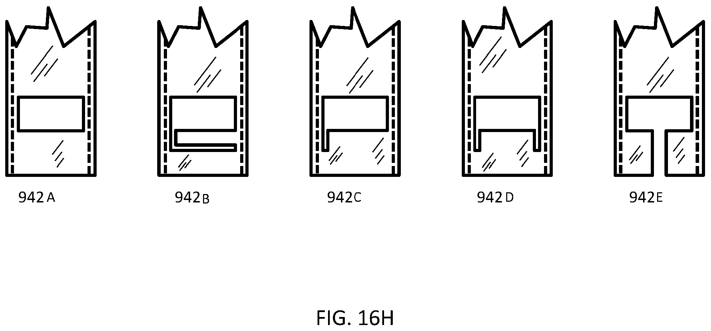

**Fig. 16H** shows schematically additional exemplary configurations of a distal region of PPM tubes for interfacing with a shuttle;

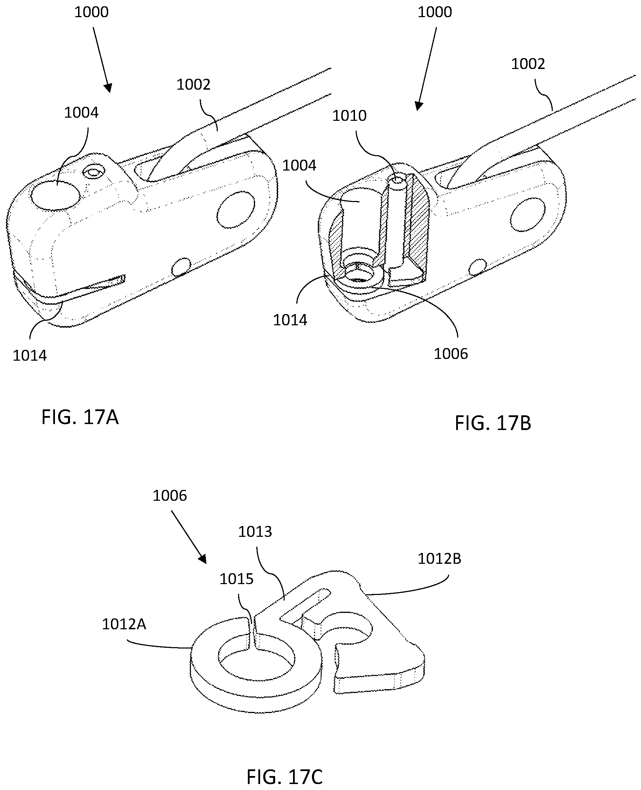

**Fig. 17A** is a perspective view of a receiver module, according to some embodiments;
**Fig. 17B** is a partially cut-away view similar to FIG. 17A;
**Fig. 17C** shows a perspective view of an engagement element from the receiver module of Fig. 17A;

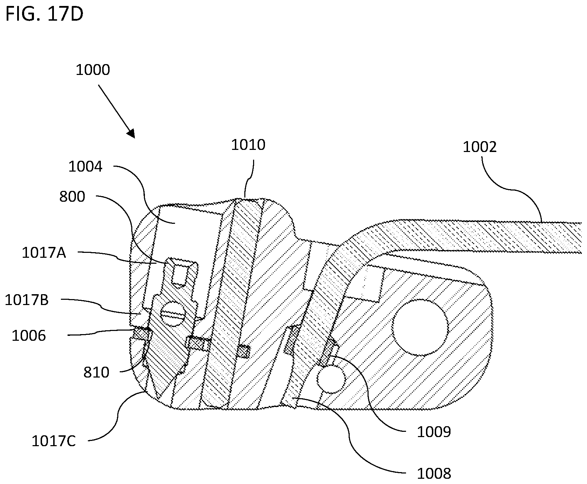

**Fig. 17D** shows a cross-sectional view taken axially through the receiver module of Fig. 17A while associated with a shuttle;

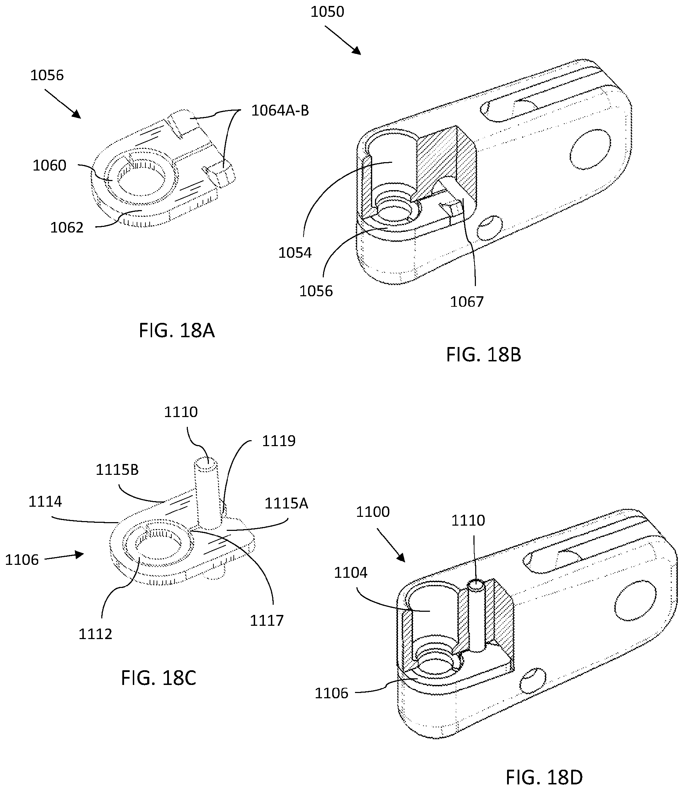

**Figs. 18A and 18C** are perspective views of alternative implementations of a shuttle engaging element according to variant implementations of an embodiment of the present invention;
**Figs. 18B and 18D** are partially cut-away perspective views of receiver modules employing the shuttle engagement elements of Figs. 18A and 18C, respectively;

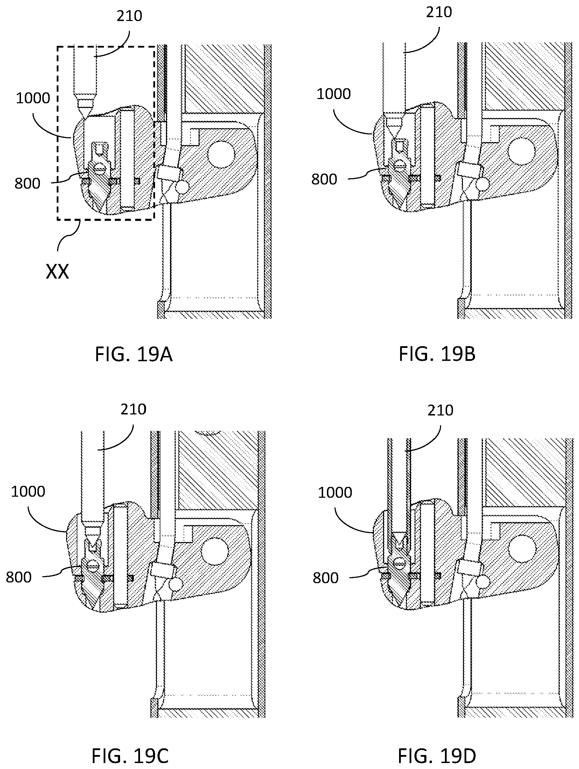

**Figs. 19A-19D** are schematic cross-sectional views of an off-axis approach of a shuttle transmitter approaching a shuttle within the bore of a shuttle receiver, illustrating successive stages of a self-aligning process according to a further aspect of an embodiment of the present invention;

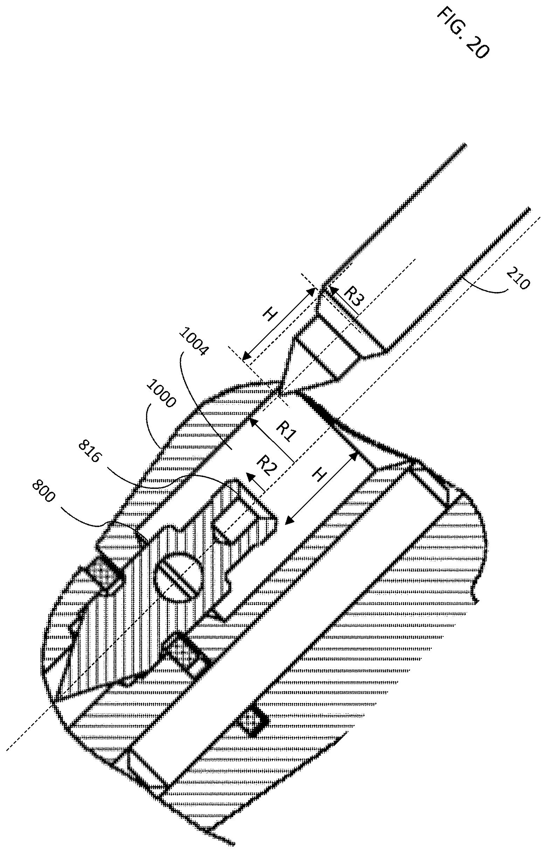

**Fig. 20** is an annotated and enlarged view of the region of Fig. 19A marked by box XX;

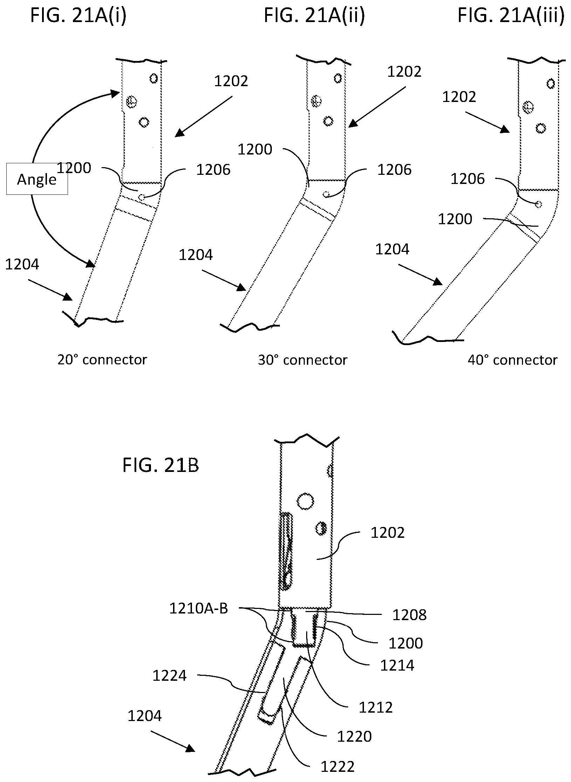

**Figs. 21A(i)-(iii)** are side views of a swivel connector, according to some embodiments, illustrating three different fixed-angle swivel connectors providing different deflection angles;
**Fig. 21B** is a partially cut-away side view similar to Fig. 21A(i);

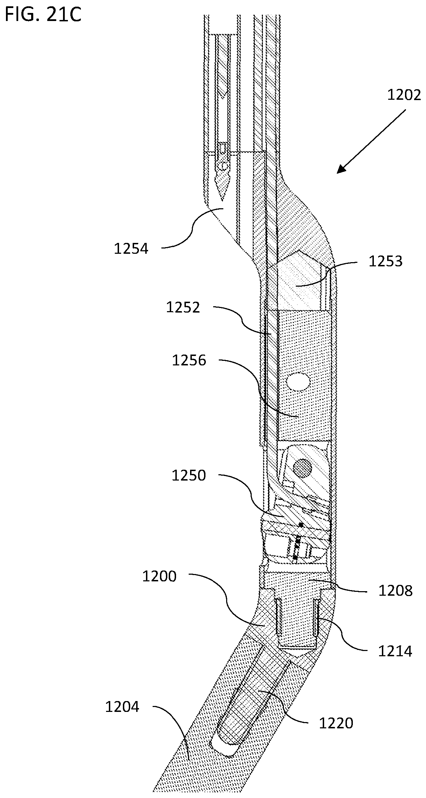

**Fig. 21C** is an axial cross-sectional view of a distal end of a suturing mechanism connected via the swivel connector of Fig. 21A(i) to a dilator;

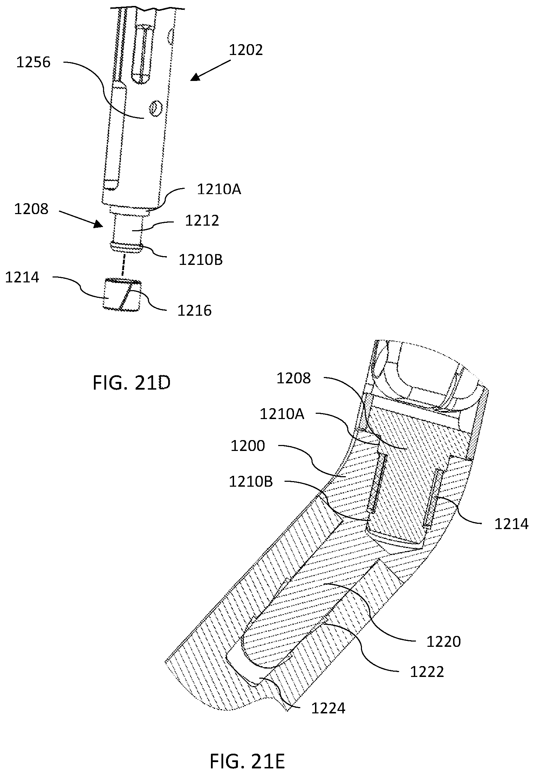

**Fig. 21D** is a perspective view of the distal end of the suture mechanism module shaft from Fig. 21C; and
**Fig. 21E** is an enlarged axial cross-sectional view taken through the swivel connector of Fig. 21A(i) associated with an insert distal end.

### DESCRIPTION OF THE PREFERRED EMBODIMENTS

The present invention provides mechanisms for operating medical devices, such as suturing devices, and various details of such devices.

By way of introduction, the present invention introduces a class of mechanisms which have utility for operating devices in a broad range of medical and other applications, wherever a combined motion is required of two elements, referred to as a first effector and a second effector. Specifically, the mechanisms address cases in which two effectors are required to perform a combined motion in which they move together and additionally need to switch between two states in which a relative motion occurs between the two effectors. An exemplary application which requires such motion is a suturing mechanism, where a first displacement may cause a needle to penetrate tissue and become lodged in a distal needle receiver (or "pocket"), and a subsequent shift between two elements may release the needle to be left in the pocket and retract. Additionally, or alternatively, a combined displacement may bring a needle holder to engage a needle that is held on a distal side of tissue, and a relative shift between two elements may cause the needle holder to grip the needle for withdrawal through the tissue. However, the mechanism is not limited per se to any particular implementation of a suturing mechanism, and may also be adapted to various other applications in the medical field and elsewhere. Accordingly, FIGS. 1A-3B will present the features of various implementations of this mechanism in a schematic manner, applicable to a wide range of applications, and encompassing multiple possible implementations.

Then, by way of one specific example, an implementation of the mechanism will be described in the context of one particularly-preferred embodiment with reference to FIGS. 4A-7G, and a number of complementary features which are advantageously implemented in a particularly preferred suturing device according to various further aspects of the present invention will be described with reference to FIGS. 8A-20C.

### Generic Actuator with Displaceable Bistable Mechanism

Referring now generically to FIGS. 1A-3B, there is shown a mechanism, generally designated **100,** constructed and operative according to an aspect of the present invention, for operating a device, such as a medical device, and according to some particularly-preferred implementations, a suturing device. Details of the device are not shown in these drawings, but will be illustrated by way of a non-limiting example later in this document. Mechanism **100** generates motion relative to an external reference, illustrated here as an external housing or handle **102.** An effector assembly **104** is mounted relative to handle **102** so as to be displaceable along an axial direction (arrow **106**) relative to the handle. Effector assembly **104** includes a first effector **184** and a second effector **182.** The effectors are the elements which interact with, or form part of, the device being actuated. In the particularly preferred but non-limiting examples illustrated below, first effector **184** is a holder for holding a suturing needle and second effector **182** is an ejector for releasing the suturing needle from the holder. Thus, throughout the document, these elements may be referred to as holder **184** and ejector **182,** although it should be noted that these terms apply only to a non-limiting subgroup of exemplary implementations of the present invention.

The effector assembly **104** further includes a bistable mechanism **170** including a bistable element **172,** and a biasing arrangement including at least a first spring element **180B** and a second spring element **180A.** The biasing arrangement biases each of the first and second effectors **184** and **182** in a distal direction relative to the bistable element **172.** As best seen in the pairs of bistable states illustrated in FIGS. 2A(i) through 2D(ii), the bistable element **172** assumes a first axial state (FIGS. 2A(i), 2B(i), 2C(i) and 2D(i)) in which the second effector **182** is biased towards a first relative axial position relative to the first effector **184,** and a second axial state (FIGS. 2A(ii), 2B(ii), 2C(ii) and 2D(ii)) in which the second effector **182** is biased towards a second relative axial position relative to the first effector **184.**

Optionally, the components of the effector assembly **104** may all be contained within an inner housing **185,** which is preferably integrated so as to move together first effector **184,** and which provides features (shown schematically as stops **108** in FIGS. 2A(ii), 2B(ii), 2C(ii) and 2D(ii)and 2D(ii)) relative to which the two axial positions of the bistable mechanism **170** are defined.

Mechanism **100** also includes a force input deployed to selectively apply an input force to bistable mechanism **170.** The force input may be manual or may be actuated by any form of electromechanical or other actuator. Additionally, the manual or otherwise-actuated force input may be unidirectional (with a reverse motion provided, for example, by a return spring), or the input may actively provide force bidirectionally. In the preferred but non-limiting examples illustrated herein, the force input is implemented as a push-button, knob or plunger **114** to which force is applied manually in only one direction, causing distal displacement of the effector assembly **104** relative to handle **102,** while the return motion is governed by a return spring **140.**

FIGS. 1A-1C illustrate different forms of deployment of the biasing arrangement, and in particular, whether first spring element **180B** and second spring element **180A** are deployed in parallel or in series. Thus, in the example of FIG. 1A, first spring element **180B** acts between first effector **184** and bistable element **172,** while second spring element **180A** acts between second effector **182** and bistable element **172,** such that the two springs thereby defining a parallel biasing arrangement. In FIG. 1B, first spring element **180B** acts between first effector **184** and the second effector **182,** while second spring element **180A** acts between second effector **182** and bistable element **172,** thereby defining a serial biasing arrangement. FIG. 1C illustrates a hybrid arrangement in which first and second spring elements **180B, 180A** are deployed as in FIG. 1B, but they are supplemented by a further spring **120** which acts directly between first effector **184** and bistable element **172.** Additionally, in certain preferred implementations, at least one and typically both of first and second springs **180B** and **180A** are deployed with a preload force that defines a minimum force required to change a length of the at least one spring. A ratio between the preload forces of first and second spring elements **180B, 180A** typically differs between the first axial state and the second axial state of the bistable mechanism.

An arrangement for providing these preload forces is illustrated schematically in FIGS. 1A-1C by providing corresponding spring enclosures **181A** and **181B** for springs **180A** and **180B,** respectively, with input to each spring delivered via a corresponding compression rod **186A** and **186B**, respectively. This depiction of each spring in a dedicated enclosure is for clarity of illustration, but it will be appreciated that there are many ways in which a spring can be confined so as to have a preload force. Certain alternative implementations will become apparent from the subsequent examples.

All of the springs shown herein are represented schematically as helical compression springs, but it will be appreciated by a person having ordinary skill in the art that the present invention may be implemented using any and all types of spring or other resilient elements including, but not limited to, compression springs, extension springs, torsion springs and any other forms of mechanical springs formed from elastic or otherwise resilient material as discrete spring elements or integrally formed with other components, from metal, metal alloy, superplastic alloy, polymer materials or any other material exhibiting properties suitable for forming springs, as well as pneumatic springs, magnetic or electromagnetic arrangements emulating or replacing springs, and any other element or device which provides the biasing properties described herein.

According to one aspect of certain implementations of the present invention, the overall effect of the structures described herein is that a force applied by the force input in a first direction is effective first to displace the effector assembly distally along the axial direction without changing a state of the bistable mechanism, and then, when at least part of the effector assembly encounters an obstacle to further distal displacement, to toggle the bistable element between the first axial state and the second axial state. This aspect of certain implementations of the present invention will be illustrated with reference to FIGS. 2A-2D.

According to a second aspect of certain implementations of the present invention, the biasing arrangement is configured such that the bistable mechanism can be toggled from the first state to the second state without requiring the second effector to have reached the second relative axial position. This will be illustrated with reference to FIGS. 3A-3B.

Turning now to FIGS. 2A(i)-(iv), these views illustrate schematically an implementation of mechanism **100** according to the "parallel" implementation of FIG. 1A. First effector **184** is here illustrated as a holder in the form of a tube while second effector **182** is here illustrated as an ejector in the form of a pointed rod. Second spring **180A** is here confined within an enclosure **181A.** One side of enclosure **181A** abuts, is attached to, or is integrally formed with, the bistable element **172,** while the rear end of second effector **182** is formed as a compression rod acting on second spring **180A.** Thus, second spring **180A** acts between bistable element **172** and second effector **182.** Parenthetically, here and throughout this document, the phrase "acts between" relates to the functional relationship between the biasing element and the corresponding components on which it acts, but does not limit the deployment of the elements to being spatially interposed between those components. First spring **180B** is contained (and preferably preloaded) between external surfaces of spring enclosure **181A** and an internal shoulder of first effector **184** so that first spring **180B** acts between first effector **184** and bistable element **172.**

The spring constants and preloads of first and second springs **180B** and **180A** are such that a force applied to plunger **114** is effective first to displace the effector assembly distally along the axial direction against retraction spring **140** without changing a state of the bistable mechanism, corresponding to the motion from FIG. 2A(i) to FIG. 2A(ii). This motion terminates when an obstacle is encountered which provides a resistance to further motion which overcomes the preloading of the relevant springs of the biasing arrangement. Typically, when used to operate a suturing device, such resistance is incurred when the effectors encounter a needle, a needle-holding pocket or other similar structure, as will be illustrated below. Optionally, as illustrated here, a step and shoulder configuration may also be provided between the external housing or handle **102** and the inner housing **185** which defines a maximum displacement of the effector assembly relative to the handle. In either case, when an obstacle to further distal displacement is reached, further force applied to plunger **114** compresses at least first spring **180B** until the bistable mechanism toggles between the first axial state (FIG. 2A(ii)) and the second axial state (FIG. 2A(iii)). This transition occurs through compression of the mechanism beyond the states illustrated here, to reach a transition state, followed by retracting into the second state. Only the final states of the bistable mechanism when not under external strain are illustrated here. More details of the transition process and of the system's response to external forces will be addressed below. Preferably, under action of retraction spring **140** (or in alternative implementations, when a reverse actuation force is applied to the force input), the effector assembly then retracts relative to handle **102** while maintaining the new state of the bistable mechanism, reaching the state of FIG. 2A(iv). This process is reversible, such that, when the force input is next actuated, the process will repeat, this time toggling from the second state of FIG. 2A(iii) back to the first state of FIG. 2A(ii), followed by retraction back to the state of FIG. 2A(i), after which the entire process can repeat.

The bistable mechanism **170** is shown here schematically, and can be implemented as substantially any bistable mechanism which is actuated by axial force to alternately switch between two states corresponding to two axial positions of bistable element **172** relative to internal housing **185.** A wide range of such mechanisms are known, for example, in the context of retractable pens, including but not limited to, various bistable mechanisms with rotating cams, pin-in-slot or ball-in-slot bistable mechanisms, and rocker-based bistable mechanisms. Variations of such mechanisms are numerous, and their designs per se are well-known, so for conciseness of presentation, it is unnecessary to address the details of multiple examples of bistable mechanisms. One particularly preferred subgroup of bistable mechanisms are those employing a rotary cam with axially-directed sloped surfaces which is slightly rotated by actuation surfaces of a sliding actuator, and which has projections which successively settle into slots in housing **185** defining two different axial positions. One such non-limiting example will be described below in detail.

Particularly preferred bistable mechanisms are often referred to as "click mechanisms" due to the distinctive audible and/or tactile feedback ("clicking") which is generated, typically twice, during each transition.

Optionally, some part of the bistable mechanism may be attached to the end of plunger **114.** It is noted, however, that such attachment is not necessary. FIG. 2A(iv)' illustrates a state similar to FIG. 2A(iv) in which part of bistable mechanism **170** can move independently from both the bistable element **172** and from plunger **114,** as illustrated by the space on either side of the element labeled **170.**

Although the bistable mechanism per se is analogous to mechanisms employed elsewhere, the manner in which it is used according to embodiments of the present invention differs in many respects from common applications of such mechanisms. The bistable mechanism **170** is used herein to generate relative displacement between two effectors which are themselves displaceable as a unit relative to handle **102.** Most preferably, a single force input is employed to generate sequentially both the combined motion of the effectors and the toggling of their relative positions. The biasing arrangement also provides distinctive functionality that is not normally present in bistable mechanisms, as further detailed below.

Turning now to FIGS. 2B(i)-(iv), these are similar to FIGS. 2A(i)-(iv), but illustrate a series deployment of the biasing arrangement. Thus, in this case, first spring **180B** acts between first effector **184** and second effector **182,** while second spring **180A** acts between second effector **182** and bistable element **172.** Structurally, in this non-limiting example, this is achieved by attaching second effector **182** rigidly (or integrally formed) with second spring enclosure **181A,** and providing an actuator rod **186A** that links the bistable element **172** to provide an input to second spring **180A.** First spring **180B** is trapped between spring enclosure **181A** and an internal shoulder of first effector **184.** Both springs **180A** and **180B** are preferably preloaded such that, as before, force applied to the force input (plunger **114**) initially generates displacement of the entire effector assembly without changing the state of the bistable mechanism (FIG. 2B(i) to FIG. 2B(ii)), and then, after encountering an obstacle (either the step in outer housing/handle **102** or an external obstacle such as a needle-receiving pocket), further force causes toggling of the bistable mechanism (FIG. 2B(ii) to FIG. 2B(iii)), via a transition state not shown. Withdrawal then takes place to the state of FIG. 2B(iv), either under the effect of retraction spring **140** or through positive application of a withdrawal force, all according to details of the specific implementation.

FIGS. 2C(i)-(ii) illustrate an alternative implementation with a series deployment of the biasing arrangement functionally equivalent to the implementation of FIGS. 2B(i)-(iv) while being structurally more similar to the implementations of FIGS. 2A(i)-(iv). For conciseness, only the states before and after toggling of the bistable mechanism, corresponding to FIGS. 2B(ii) and 2B(iii), are illustrated here. The structure shown here is almost identical to that of FIGS. 2A(i)-(iv) except that first spring **180** is here delimited on one side by a flange **110** projecting from second effector (ejector) **182,** so that the spring acts between the first effector **184** and second effector **182.** This defines the biasing arrangement as a series deployment, functionally similar to FIG. 1B and FIGS. 2B(i)-(iv).

FIGS. 2D(i)-(ii) illustrate a further alternative implementation employing a hybrid implementation of the biasing arrangement, where a series structure similar to that of FIGS. 2B(i)-(iv) is supplemented by an additional spring **120** which acts directly between first effector **184** and bistable element **172.**

The distinctions between the various options of parallel, series or hybrid deployment of the biasing arrangement typically do not greatly impact the operation of the device as experienced by a user, but can be significant in defining the properties required for each spring, how sensitive the design is to manufacturing tolerances, and how much force the user needs to exert in order to toggle the state of the bistable mechanism. Additional design considerations for achieving the required device functionality in certain applications will be discussed further below.

FIG. 3A illustrates in more detail the operation of the device of FIG. 2A in a scenario of a suturing device (suture thread not shown) in which the mechanism delivers a needle **800** into a pocket **1000** and releases the needle before withdrawing (sequence stages 1-7), and subsequently extends to engage the needle and withdraw it from the pocket (sequence stages 8-14). All parts of the structure, including the needle and the pocket, are illustrated here only schematically. Particularly preferred non-limiting examples of specific implementations of these elements will be discussed below.

Turning first to FIG. 3A(i), the transition from stage 1 to stage 2 is similar to the transition from FIG. 2A(i) to 2A(ii), except that in this case, the motion is effective to deposit needle **800** into pocket **1000**, and the pocket then provides an obstacle to further motion of at least the first effector (holder) **184.** Further applied force overcomes the preload force of first spring **180B**, which compresses until second effector (ejector) **182** contacts the needle **800** (stage 3). At this point, both the first and second effectors are obstructed from moving further, so further force applied to the input plunger causes compression of both first and second springs **180B**, **180A** until the plunger forces the bistable mechanism into a transition state (stage 4, typically generating a first "click") and, on partial release of the force on the plunger, the bistable mechanism settles into the second state, with bistable element **172** lodged in a distally-displaced position relative to holder **184** (stage 5, typically generating a second "click"). Unlike conventional applications of bistable mechanisms, due to the presence of second spring **180A**, the bistable mechanism is here able to transition to its second state despite the fact that the second effector (ejector) **182** has not yet reached its forward-displaced position relative to first effector (holder) **184.** In this state, second spring **180A** biases ejector **182** forwards relative to holder **184** so that ejector **182** ejects needle **800** from holder **184** simultaneously with a slight rearward motion of the holder (stage 6). Ejector **182** then assumes its fully extended position and retraction of the effector assembly is completed (stage 7). This completes the process of transferring the needle from the holder to a needle receiver and withdrawing the holder. This process, when performed through one or more layers of tissue using a needle that carries with it a suture, is effective to perform a proximal-to-distal stitch through the tissue.

In order to complete a running stitch, both the needle receiver that is holding the needle and the effector assembly are preferably relocated so as to be aligned with each other on opposite sides of a second location of tissue, for the distal-to-proximal stitch process illustrated in stages 8-14.

From stage 8, by pressing on plunger **114,** the effector assembly is advanced while the bistable mechanism is in its second state, with ejector **182** projecting. In this state, the ejector most preferably provides a penetrating point, allowing the effector assembly to penetrate through tissue to reach the needle and needle receiver. (The mechanism is also applicable to other implementations in which a double-ended needle penetrates the tissue from the distal side, in which case no penetrating point is required on the effector assembly.) Once ejector **182** contacts the needle, and is thus obstructed from advancing, further force applied to the force input compresses second spring **180A** (stage 9) while holder **184** continues to move forward via force conveyed through first spring **180B** until holder **184** engages needle **800** (stage 10). Further forward force at this stage compresses first and second springs **180B, 180A** until the plunger forces the bistable mechanism into a transition state (stage 11, typically generating a "click") and, on partial release of the force on the plunger, the bistable mechanism returns to the first state, allowing bistable element **172** to be retracted relative to holder **184** (stage 12, typically generating a further "click", followed by further retraction at stage 13). The engagement of holder **184** with needle **800** is such that retraction of the effector assembly is then effective to free needle **800** from needle receiver **1000** and withdraw the needle through the tissue (not shown), thereby completing a distal-to-proximal suture stitch (stage 14).

By relocating mechanism **100** and needle receiver **1000** to successive locations and repeating sequence 1-7 in one location and then 8-14 in another location, it is possible to form a running suture stitch through tissue.

Based on the desired sequence of operations described in FIG. 3A, it is possible to define various properties of the springs and other force-related components to ensure proper operation of the sequence. For example, in this implementation, at least some and preferably all of the following criteria are preferably met:
- Retraction spring **140,** where used, should be sufficiently strong to withdraw the effector assembly (in either configuration, with or without the needle) through any tissue likely to be encountered.
- First and second springs **180B, 180A** should have sufficient preload force to allow penetration of any soft tissue or semi-hard tissue that may be encountered during penetration, in order to avoid toggling the bistable mechanism before reaching the needle receiver. (It should be possible to avoid encountering bone or other hard tissue by proper pre-operative planning and/or use of intra-operative imaging technology.)
- The force exerted by first spring **180B** should be sufficient to engage holder **184** with needle **800.**
- The force exerted by second spring **180A** should be sufficient to eject the needle from holder **184.**
- The needle retention force of holder **184** should be greater than the needle retention force within the needle receiver ("pocket") **1000.**

FIG. 3B is equivalent to FIG. 3A(i) but shows the series bias arrangement of FIG. 2B. The sequence is essentially similar to that described above, but differs in the specific interrelationships required between the preload forces and spring constants for each spring to provide the required operations. These different options provide different degrees of freedom in the system design, which may relax certain design constraints, thereby facilitating manufacture and/or ensuring enhanced reliability of the device.

### Suturing Device Application

The remainder of the description relates to one particularly preferred but non-limiting example of an application of the above mechanism as applied to a particularly advantageous suturing device. The disclosure will also relate to various features of the suturing device which are believed to be of patentable significance in their own right, independent of the actuation mechanism used in the device.

According to some embodiments, the present disclosure relates, inter alia, to various aspects of components of a suturing device, comprising, operating handle (user interface handle), needle (shuttle), displacement module, bleeders, transmitter module, which are particularly suited although not necessarily limited, to use with a suturing mechanism such as that described in PCT patent application nos. PCT/IB2020/057513, and PCT application No. PCT/IB2020/061610.

According to some embodiments, the suturing devices and methods disclosed herein relate to suturing a tissue (such as, blood vessel walls), as part of a surgical procedure. According to some embodiments, the devices and methods disclosed herein are applicable to a variety of medical procedures, including, for example, but not limited to external, superficial, shallow incisions, minimally invasive procedures, surgically procedures and structural heart related procedures, such as Patent foramen ovale (PFO). In some exemplary embodiments, the devices and methods disclosed herein are for use in vascular closure procedures.

According to some embodiments, as detailed herein, the suturing device includes an operating handle, a shuttle (also referred to herein as 'needle") capable of having various shapes/forms, as detailed below, but generally in the form of a pointed arrow at its distal end and a proximal, truncated arrow-shaped end, having an axial dent/hole, and a shuttle transmitter module (also referred to herein as "needle transmitter module", "Push-Pull Mechanism" or "PPM"), which is configured to selectively displace, hold and release the shuttle. The shuttle transmitter is configured to manipulate the shuttle from a first side of the tissue (for example, vessel wall), to allow passes of the suture from the first side to a second side and from the second side to the first side. The shuttle transmitter module may include at least two elements: a shuttle ejector (also referred to herein as "releaser element", "shuttle releaser", "needle ejector" or "needle releaser"), corresponding to the second effector **182** described above, and a shuttle holder (also referred to herein as "needle holder", or "tube"), corresponding to the first effector **184** described above, wherein the shuttle holder is configured to hold/associate with the shuttle, and the shuttle ejector can release the shuttle from the holder. In some embodiments, the shuttle holder may be implemented as a tubular or essentially tubular element that can engage with an external region/surface of the shuttle, while the releaser element may be implemented as a rod, which may in some embodiments, be displaceable internally within the tubular element. The releaser element is preferably pointed at its distal end, optimally having a distal penetrating end shaped for penetrating through a tissue when protruding from the shuttle holder (PPM tube).

According to some embodiments, as further detailed herein, the shuttle may be held and/or displaced by a shuttle receiver module (also referred to herein as "needle receiver module" or "pocket"), which is passively configured to receive, retain and release the shuttle. In some embodiments, the pocket is configured to be reversible retracted from a "closed" position to an "open" position, wherein the transition between the states may be controlled by the user, via the operating handle, as further detailed herein. In some embodiments, the shuttle transmitter module and the pocket are positioned on opposing sides of the tissue to be sutured (for example, when the pocket is inside a blood vessel, and the shuttle transmitter module is external thereto) and they may be aligned on such opposite sides by mechanical interconnection between the two modules.

In some embodiments, the shuttle, typically in the form of a pointed needle, is configured to hold a suture, and the shuttle transmitter module is configured to selectively hold and release the shuttle. When holding the shuttle, the transmitter module forms a first penetrating configuration, and after releasing the shuttle (to be held by the pocket), presents a second penetrating configuration. At this second penetrating configuration the shuttle transmitter is configured to present a pointed, needle like, distal end (in particular, the ejector element). The shuttle transmitter module can thus manipulate the shuttle from one side of the material to be sutured (for example, blood vessel or other tissue), to perform passes of the suture from one side of the tissue, to the other side of the tissue, and vice versa. To this end, a pass from the first side to the other (second) side is performed by advancing the shuttle transmitter in the first penetrating configuration, (while being held), and then facilitating releasing the shuttle from the shuttle transmitter, to be passively and temporarily held by the shuttle receiver (which is located on the opposite side of the tissue), and withdrawing the shuttle transmitter from the tissue, without the shuttle. A pass of the suture from the second side to the first side is performed by advancing the shuttle transmitter in the second penetrating configuration, (i.e., without the shuttle), for collecting and retrieving the shuttle through the sutured tissue. The shuttle transmitter is configured to penetrate the material at a second location, which is aligned with the shuttle that is temporarily retained in the pocket, engages and holds the shuttle, and withdraws the shuttle through the tissue at the second location. During each pass, the shuttle draws with it the suture such that the suture extends into the tissue at the first location and out of the material at the second location. Such a suturing process can thus be used for a variety of procedures, including, for example, but not limited to: closure of external shallow incisions, closure of superficial shallow incisions, minimally invasive procedures, conventional surgical procedures, coronary procedures, cardiovascular procedures, vascular openings/holes closure, closure of opening between tissues (such as, for example, opening in the wall between the right and left upper heart chambers (PFO), closure of incisions, closure of wounds, attachment of two or more materials arranged in overlapping relation by suturing through both layers; bringing together of two edges of two regions of material, anchoring of a suture in a material by forming stitches in overlapping relation by repeated closely-adjacent passes through the material, suturing to interconnect a prosthetic device or material with natural tissue, where the material may be a natural biological tissue or any other material, Patent foramen ovale (PFO), Atrial septal defect (ASD), Left atrial appendage occlusion (LAAO), Left atrial appendage closure (LAAC), Aneurysm repairs, transcatheter valve repairs, minimally invasive heart apex closure, a minimally invasive repair of left ventricular, suturing operations during endoscopic procedures, laparoscopic procedures, gastroscopic procedures, otoscopic procedures and minimally invasive gynecologic procedures, and the like, or combinations thereof. Each possibility is a separate embodiment.

Reference is now made to **Fig. 4A**which schematically depicts a perspective view of a suturing device, according to some embodiments. As shown in Fig. 4A, suturing device 10, includes a handle portion 12, having operation button (also referred to herein as "suture button") 14, at a proximal end thereof, and a rotation interface 16, facilitating axial rotation of the handle by the user (for example, by the holding palm of the user). Rotational interface 16 may optionally comprise one or more indicators 18 thereof, aiming to guide and aid a user in operating the device, by, for example, indicating, pocket operation (deployment or retraction thereof), radial positioning of the device, in accordance with the suturing operation, and the like. In some cases, rotation interface 16 may simply be a flange to facilitate manual grip and manipulation, as illustrated in FIG. 4C. Further shown in Fig. 4A is removable safety catch 20, which is configured to lock/prevent the activation of operating button 14 when in a closed position, and to allow such operation, when released to an open position (for example, only once the pocket has been deployed, as detailed below herein). Further shown is medial handle portion body/casing/housing 22, which encloses, inter alia, operating module (a click mechanism). As exemplary shown in Fig. 4A, interface 16 may preferably have flat torus shaped geometry, forming a rounded flange around the handle portion, allowing one hand operation of button 14, while allowing the rotation of the handle within the palm of the user. Suturing device 10 may further comprise one or more indicators indicative of the positioning of the suturing needle with a tissue, more particularly within a blood vessel. Further shown in Fig. 4A are indicators 24A-B, exemplified in the form of bleeders, configured to allow blood flow from the suturing region towards the bleeders. One of the bleeders (for example, 24A) may serve as a "GO" bleeder (i.e., when blood is flowing via the bleeder, the distal end of the suturing device is properly positioned (sufficiently inserted) with the target tissue); and the other bleeder (for example, 24B), may serve as a "NO-GO", bleeder (i.e., when blood is flowing via the bleeder, the distal end of the suturing device is not properly positioned, and specifically, over-inserted). Additionally, at a distal region thereof, handle 12 further comprises a pocket deployment mechanism (cam) 26, which is configured to allow the opening (deployment) or closing of the pocket (needle receiver module), of suture mechanism 30, which is located in a more distal region of shaft 32, interconnecting the distal end of handle portion 12, and the suture mechanism 30. Suturing device 10 may further optionally comprise a dilator 34, connected to a distal end of the shaft, via an optional fixed angle swivel connector 36, and may be used to aid in dilating target tissue, allowing the insertion or removal of the shaft, suturing mechanism, or any other medical tools used during the medical procedure.

Reference is now made to **Fig. 4B****,** which schematically illustrates a close-up perspective view of a cross section of a handle, according to some embodiments. As shown in Fig. 4B, handle 12, includes operating button 14, configured to move longitudinally (up-down), as initiated by pressing (pushing) the button by a user. Each press/push of the button actuates a mechanism, such as that described above with reference to FIGS. 1A-3B, generating displacement of the shuttle transmitter and toggling between different internal states, which ultimately drives the suturing mechanism, as will be described in detail below herein. Further shown is internal retraction (return) element 40, which may, in some exemplary embodiments, be a retraction spring, configured to allow retraction of the operating button, and associated click mechanism, during operation thereof. Further shown is interface 16, which as exemplary shown in Fig. 2, may have an essentially flat torus shaped geometry, allowing one hand operation of button 14, while facilitating the rotation of handle 12 within the palm of the user, as illustrated in Fig. 4C. In addition, operating module (also referred to as click mechanism module) 50, corresponding to the effector assembly described above, is located within the internal casing of handle 12. The click mechanism module, the specific embodiment of which will be discussed in detail below, includes a combination of internal springs/spring like elements as well as elements of a bistable mechanism (linear and/or circular), and is used to enable the suture state machine steps, while preventing overloading of the needle receiver module (i.e., preventing the exertion of too much force on the pocket, to prevent distortion or breakage thereof) and advantageously while providing tactile feedback to the user. Further shown is tube and ejector rod 52 of the shuttle transmitter module, which is connected on the proximal end to the click mechanism module and extends longitudinally along the distal end of the handle and through shaft 34, to the suturing mechanism. Also shown in Fig. 2 are bleeders 24A-B, and pocket deployment mechanism 56, which is activated by pocket deployment mechanism knob 26 (shown in Fig. 4A).

Fig. 4C schematically depicts a perspective view of handle 2 of a suturing device while being held in the palm (15) of a user; according to some embodiments

Reference is now made to **Figs. 5A-5C****,** which show additional details of an exemplary click mechanism module, according to some embodiments. Fig. 5A shows the interior of one side of housing 22 and the click mechanism module 50 removed therefrom. It may be seen that housing 22 includes one or more elongated axial slot 23, while click mechanism module 50 features a corresponding projection 25 for sliding engagement within slot 23, thereby allowing click mechanism module 50 to slide axially within housing 22 without rotating about the axis. As shown in the partially cut-away view of Fig. 5B, click mechanism module 50 is made of a housing (envelope) and may be comprised of at least two portions (60A-B) that may associate together, transiently or permanently, by any suitable means (such as, screws, adhesives, attachment elements, welding, and the like). The housing can be made of any suitable materials, including, for example, plastic, metal, aluminum, and the like. The housing may include one or more structures that may be used in the operation of the click mechanism module, such as, for example, opening 62A and fixed guide surfaces 64, that may interact with one or more internal elements of a bistable mechanism, as detailed below. The bistable mechanism is also referred to herein as a "toggle mechanism" due to its ability to toggle between two stable states. Click mechanism module 50 includes a combination of internal elements, as well as a combination of springs, that allows toggling between different stable states at the press of an operating button. Shown in Fig. 5B is a reciprocating toggle shaft 70, which is connected at a proximal end thereof to an operating button (not shown), and at a distal end thereof includes or is associated with a bistable mechanism actuator element (toggle teeth) 72, which is configured to interact with rotatable bistable element (cam) 74. Rotatable bistable element 74 in turn can define different internal states (for example, state A and state B), of the suturing mechanism, by virtue of its relative position, and changes of internal flexible elements (such as springs), configured to drive movement of elements of the suturing mechanism (in particular, the shuttle transmitter module elements). As shown in Fig. 5B, the click mechanism module includes a combination of flexible elements, such as, springs, that can operate in series or in parallel (as detailed herein-above), wherein each set of flexible elements can interact or activate/control movement of a different element of the suturing mechanism, in particular, elements of the shuttle transmitter module. For example, flexible element (spring) 80A is configured to interact/activate/control movement of the shuttle ejector element (shuttle releaser) of the shuttle transmitter module, and flexible element (spring) 80B is configured to interact/activate/control movement of the shuttle holder element (shuttle releaser) of the needle transmitter module, which is directly associated/connected to the toggle mechanism envelope (casing). In some embodiments, the relative size, diameter, force, flexibility and/or any other property of the flexible elements can affect the relative movement of each of the associated suturing mechanism elements, and the interplay between the flexible elements facilitates such control. In some embodiments, the relative position between the toggle elements (of the reciprocating and rotatable toggle mechanisms) and the fixated toggle elements (of the envelope of the click mechanism) can affect the forces exerted on the insertion module elements. Further shown in Fig. 5B is shuttle ejector collet 82, and shuttle releaser collet 84. Thus, as illustrated in Fig. 5B, click mechanism 50 can enable the smooth and reliable operation of the suturing mechanism.

According to some embodiments, the bistable mechanism is configured to provide tactile and/or audible feedback to the user, at the end of each press (inward motion), e.g., for insertion or retrieval of the PPM and the shuttle. This advantageously allows the user to be certain that the mechanism has properly changed states during each stage of the suturing process.

Reference is now made to Fig. 5C, which shows a cross section of a handle having a click mechanism according to some embodiments. As shown in Fig. 5C, handle 12 includes a click mechanism module 50. Click mechanism module 50 includes a combination of toggle elements, as well as a combination of springs, that allows toggling between different states at the press of the operating button 14. Shown in Fig. 5C is reciprocating toggle shaft 70, which is connected at a proximal end thereof to operating button 14, and at a distal end thereof is associated with sliding bistable mechanism actuator element (toggle teeth) 72, which is configured to interact with rotatable bistable element (cam) 74, and drive a rotary bistable mechanism, that can move axially between the different states

According to some embodiments, the advantageous click mechanism can transition between states A and B, in accordance with the toggle mechanism action. As detailed herein, State A is configured to realize a sequence of states for transferring the shuttle from the transmitter module to the receiving module (pocket). As detailed herein, the transfer of the shuttle from the transmitter module to the pocket is performed through the material being sutured State B is configured to realize a sequence of states for transferring the needle from the receiver module (pocket) to the transmitter module (insertion module), while the ejector element protrudes from the holder (tube), while being configured to present a pointed, needle like, distal end capable of penetrating the material. In order to better understand the states and the steps involved, reference is now made to **Fig. 6****,** which schematically illustrates states of the suturing mechanism, during the performance of a suture, according to some embodiments. Shown in Fig. 6 are general states (0-7), of a suturing mechanism, showing the position of the shuttle at each of the states of insertion thereof (by the transmitter module), and the passively receiving thereof (by the pocket). Shown in Fig. 6 are the elements of the shuttle transmitter module (ejector and holder), and the receiving module. At state 0 (before insertion, or after the end of a suturing cycle), the shuttle 200 is held within the transmitter module in a retracted position, ready to be deployed. Pocket 202 is in a closed (retracted) position. At state 1, i.e, after positioning of the suturing mechanism in the target tissue, the pocket is deployed/opened (for example, within a blood vessel), while the needle is still in a retracted position. Further shown is ejector 212 and holder 214 of transmitter module 210. At state 2, the needle is inserted by virtue of the lateral movement of the transmission module (PPM - push pull mechanism) module, traverses the tissue (not shown) and interacts with the pocket (deployed in the second side of the tissue). At state 3, ejector element 212 associates with the shuttle (more particularly, with the proximal end thereof), as detailed herein. At states 4-6, the shuttle relative position does not change, however, the toggle mechanism (located in the handle) is configured to change positions/states (from state A to state B), and the insertion mechanism retracts, and the needle is ejected therefrom. At the end position at state 7, the shuttle, which has been released from the insertion mechanism, is associated with the pocket, and the transmitter module is retracted, ready for another cycle of suturing. When reactivated, the click mechanism, being in state B, can affect states 7 to 0 (in reverse order), to enable the movement of the transmitter module from the retracted (proximal) position, to allow the ejector element to protrude out of the tube, penetrate the tissue, and enable the association of the shuttle with the holder element (tube) of the transmitter module, and release (pull) thereof from the pocket.

Reference is now made to **Figs. 7A-E,** which show schematic cross-section of the handle including a click mechanism module, in 7 different states corresponding to states 1-7 of both FIG. 6 and of the schematic illustrations of FIG. 3A(i), according to some embodiments. Reference is now made to **Fig. 7A****,** which shows a cross-section of the handle at an initial position of the click mechanism state machine. Shown is handle 12, having operation button 14, capable of being moved longitudinally to activate the click mechanism module, and return element (retraction spring) 40. The click mechanism is shown at A state position of the toggle mechanism 50. Further shown is flexible element (second spring) 80A, configured to interact and induce movement of ejector element 82 of the insertion module. Also shown is flexible element 80B (in the form of a spring) configured to interact with holder element 84 of the transmitter module, which is associated with the envelope 85 of the click mechanism. As detailed above herein, pressing the operating button (suture button) 14 will implement the state machine sequence from 1 to 7. Shown in **Fig. 7B** state 2, in which the shuttle (not shown) is associated with (held within) the pocket (not shown). Shown in **Fig. 7C** is state 3, in which the distal end (not shown) of ejector element 82 interacts (hits) the needle (at a proximal end thereof). Shown in **Fig. 7D** is state 4, which induces the toggle action, in which the toggle switches from state A to state B (50'). At this state, the user can release the pressure on the operating button 14, thereby finalizing the toggle action. finalizing the toggle action. Next, shown in **Fig. 7E** is state 5, in which the click mechanism is locked by virtue of locking engagement 360. State 6 is shown in **Fig. 7F****,** in which the insertion module is retrieved, and the needle (not shown) is completely ejected therefrom, while remaining in interaction with the pocket (not shown). Shown in **Fig. 7G****,** is state 7, at the end position, in which the insertion module is completely retrieved (retracted), by virtue of the retraction spring 40 returning the click mechanism (and the associated operating button) to start position, while the ejector element is in a penetrating position, protruding from the holder. In this position, the click mechanism is at state B, and re-pressing the operating button, will now allow activating states 7 to 1 (i.e., in reverse order), to allow the transmitter module to be pushed, via the tissue, by virtue of the ejector element acting as a penetrating moiety (as detailed herein), towards the shuttle, while allowing the pointed distal end of the ejector element to interact with the corresponding opening in the proximal end of the shuttle, to facilitate the holder element interaction/holding with the shuttle proximal region, to pull the needle from the pocket, and to return to a starting position.

According to some embodiments, the advantageous click mechanism disclosed herein is capable of realizing a state machine for implementing repeating stiches with the suture mechanism. According to some embodiments, the click mechanism disclosed herein includes a plurality of flexible elements, such as, springs, and one or more toggle modules. In some exemplary embodiments, the click mechanism may include a plurality of springs, preferably 3 springs, such as, an ejector spring, a holder spring and a retraction spring, wherein the springs are affected by a linear toggle module.

According to some embodiments, the toggle module of the click mechanism is further capable of providing/producing tactile feedback, when toggling between states (for example, between state A and B and vice versa).

According to some embodiments, as detailed herein, the click mechanism is configured to acquire two states: A state and B state, that are interchanged by the toggle module. The A state is configured to realize a sequence of sub states for transferring the shuttle from the transmitter module to the receiver module and the B state is configured to realize a sequence of sub states for transferring the Shuttle Needle from the receiver module to the transmitter module. According to some embodiments the linear toggle module is configured to switch from A state to B state, or vice-versa, from B state to A state, at each of the end motion of the transmitter module from a proximal position to a distal position, and vice versa (i.e., when being inserted or retrieved).

### Pocket Deployment Mechanism

According to some embodiments, the suturing device disclosed herein includes a pocket deployment mechanism, facilitating the deployment (i.e., opening) of the needle receiver ("pocket") at a distal region of the shaft, while controlling said deployment via the handle of the suturing device. According to some embodiments, the pocket deployment mechanism may include a cam and closed cam follower, capable of producing tactile feedback and preferably providing a locking action at both end points of its rotation. The cam may be activated by a connected knob, or any other suitable element.

Reference is now made to **Fig. 8A****,** which schematically shows a perspective external view of a pocket deployment module, located in the handle of the suturing device, according to some embodiments. As shown in Fig. 8A, the pocket deployment module 400 is located within a distal region of the handle. The pocket deployment module includes an external operating knob 402, configured to allow operating the internal components of the pocket deployment module to allow the transitioning of the pocket from a retracted to a deployed position, and vice versa, as detailed below.

Reference is now made to **Fig. 8B****,** which shows a cut-away view of the pocket deployment module of Fig. 8A. As shown in Fig. 8B, rotatable cam 404, which is configured to connect to knob 402 (Fig. 8A), is configured to drive, by virtue of its rotation, a connecting element, that is connected at a proximal end thereof to a pivot point, and to the pocket at a distal end thereof. Further shown in Fig. 8B is preloaded spring 408, which is configured to retract or extend, in accordance with the movement of the cam, to thereby provide tension to the connecting element. Also shown is a portion of the insertion module 440 of the suturing mechanism. Preferably, the cam and connected knob are connected such that no relative movement (slipping therebetween) occurs.

Reference is now made to **Fig. 8C****,** which shows a schematic cross section of a pocket deployment mechanism in a handle of a suturing device, according to some embodiments. Deployment mechanism 400, includes rotatable knob 402, which is connected to cam 404. Cam 404 can rotate between two end positions to facilitate movement of the corresponding cam follower 420, wherein the end points are determined/defined by a groove/slot in the knob, and a corresponding protrusion on the housing/casing of the handle (as illustrated below herein). The deployment mechanism further includes a preloaded spring, which is connected to a spool element (clamping block) 414. Clamping block 414 includes core 416A and face 416B. The core may include a slit or channel, allowing the passage of the connecting element 406 therewithin. In some preferred embodiments, the connecting element may be realized from super-elastic alloy, preferably from Nitinol wire. The clamping block may be used to hold the connecting element and to further adjust its length, locking the length after device-specific calibration by tightening a clamping screw 422, which is preferably accessible from outside the handle to allow calibration adjustment after assembly. The core may be placed with the hollow space of spring 408 and the face may be connected to a top region of the spring, such that changes in the spring tension cause axial movement of the clamping block, to induce movement (for example, by stretching or releasing) of connecting element 406, to thereby induce movement (deployment or retraction) of the pocket. Such an advantageous setting allows controlling/limiting the pulling forces of the pocket, while being deployed. In some embodiments, the clamping block structure may be assembled with the deployment module using screws, snaps, clips and the like. In some embodiments, the clamping block may be secured by two separate portions of a cam follower that are configured to connect/close around the clamping block. In some embodiments, the clamping block may be assembled by snaps, circlip or by dividing/separating the cam follower into two parts and closing the two parts over the clamping block. Further shown in Fig. 8C are optional attachment elements 424, which are used for attaching/securing, in some embodiments, of the spool structure and/or the spring to the deployment mechanism body. Further shown in Fig. 8C are ejector element 442 and holder element 444 of the insertion module.

According to some embodiments, the pocket deployment mechanism may thus provide two locked over the center (OTC) positions, at the end of rotation motions thereof, such that reaction forces tend to urge the cam towards its end position rather than reversing its motion. One OTC position is for the pocket deployment and the other is for the pocket retraction. According to some embodiments, the preloaded spring associated with the clamping block structure can be used as a compensation for the connection element pull functions and for assembly tolerance. Such OTC positions are illustrated in **Fig. 8D(i)-(ii)**, which shows OTC position 480A, for pocket retraction (spring 408 in a relaxed state) and OTC position 480B, for pocket deployment (spring 408 in contracted state).

According to some embodiments, the pocket may thus be deployed or retracted (concealed) by rotating the deployment knob, for example at 90 degrees. In some embodiments, the rotation of the knob induces rotation of the cam, which can be translated between different positions, to thereby allow movement of the connecting element. In some embodiments, the deployment knob may provide tactile or visual indication regarding its position, and hence, the position of the pocket. For example, as illustrated in Fig. 8E(i)-(ii), the knob 402 may comprise openings 411A-B at a face thereof, such that the color identified in the openings may change in accordance with a closed (concealed) (411A-B) or open (retracted) position of the pocket (411A'-B'). In some embodiments, as shown in Fig. 8F, ends of the cam rotation motions may be defined by a groove/slot 413 in the knob 402, and a corresponding protrusion 415, on the housing/casing of the handle, or some other mechanical engagement for limiting the range of rotation.

According to some preferred embodiments, the connecting element for pocket deployment may comprise a super elastic material, such as, for example, a wire, preferably Nitinol wire, which can be translate between pulled and pushed states (in accordance with the OTC states). In some embodiments, to prevent buckling, the connecting element (such as, wire) may be at least partially supported by a support tube, typically threaded over the wire section passing through the deployment module. Such a support tube may be made of any suitable material, such as, plastic, metal, etc. In some embodiments, the use of a support tube may be of particular importance when in the lower OTC position, which typically includes pushing the connecting element (for example, nitinol). The use of supporting tube may thus aid and improve the pushing process. Reference is now made to **Fig. 8G****,** which illustrates such an embodiment. As shown in Fig. 8G, connecting element 404, shown as nitinol wire, is positioned within supporting tube 410. The support tube may run parallel to, in close proximity to the suturing mechanism 210.

In some embodiments, the cam follower assembly may include a cam having an indentation, groove or a slot, which is configured to interact with corresponding engagement elements (such as, bump(s), ramp(s), and the like) located on the cam follower, in close proximity to the end of the possible end rotations (OTCs). Such a setting facilitates providing improved tactile feedback to a user, at the end of the rotation of the cam, for the pocket deployment and retraction. Reference is now made to **Fig. 9A****,** which illustrates an exemplary cam and follower, allowing tactile feedback, according to some embodiments. As shown in Fig. 9A, cam 500 includes a slot 502, and follower 504 includes ramps 506A-B, which are located slightly before the extremities of the range of rotation. The form of cam 500 itself is seen more clearly in the isometric view of FIG. 9B.

According to some embodiments, the cam follower assembly of the pocket deployment module, may be in direct contact with the connecting element (as illustrated, for example, in Fig. 8C). As detailed above, the connecting element may be in the form of a wire which can behave in a spring-like manner and may be made of super elastic alloy, such as, for example, Nitinol. In such settings the connecting element may function as a spring element for compensation in the OTC tolerance and/or other tolerances (such as, assembly tolerances). Reference is now made to **Figs. 10A-C,** which illustrate an exemplary pocket deployment module, according to some embodiments. As shown in **Fig. 10A****,** deployment module 550 (housed within the handle of the suturing device) includes at least cam 552 and follower 554. Follower 554 is directly connected to connecting element 556, and by virtue of the movement of the cam between the end positions, and the corresponding linear movement of the follower, the connecting element is configured to be pulled/pushed to thereby allow corresponding retraction/deployment of the pocket (not shown). As detailed above, the rotation of the cam may be controlled by a corresponding control knob, 558. Shown in **Figs. 10B-10C** is the pocket deployment mechanism in perspective view (Fig. 10B) and cut-away view (Fig. 10C), illustrating cam 552, follower 554, connecting element 556 (for example, in the form of a super elastic material, such as, Nitinol) and the PPM shaft 560. Also shown is screw 562, configured to allow the direct connection/association between the follower 554 and the connecting element 556. In some embodiments, the screw may further be used for calibrating the push-pull mechanism of the pocket. In other embodiments, other suitable forms of connection for connecting various elements may be used, including, for example but not limited to, adhering, gluing, molding, molding-over, snaping, and the like, or any combination thereof.

### Go/No-Go Bleeder Configuration

According to further embodiments, the suturing device may include at least two bleeder assemblies, configured to provide a user indication regarding a correct/incorrect position of the suturing device (in particular, portions of the suture mechanism at the distal region thereof) with respect of the tissue in which it is deployed (for example, a blood vessel). In some embodiments, the bleeder assemblies may include two discrete assemblies, each including a discrete bleeder tube, a distal opening (outlet) and a proximal opening (outlet) that may be further connected (via a routing element (manifold)) to external bleeder tubes. In some embodiments, the bleeder assemblies are routed in the handle of the suturing device via a corresponding manifold, to connect to corresponding external bleeding tubes. In some embodiments, each of the bleeder assemblies may further include drip stop elements at or in close proximity to the end of the external dripping tube, to allow controlling/aiming/directing the blood dripping. In some embodiments, the drip stoppers function as dripping edge, dripping prevention elements, or shunt tooths. In some embodiments, one bleeder assembly is a "go bleeder", indicating to a user that if blood is dripping from an external, proximal opening, the suture mechanism is positioned correctly (sufficiently inserted) in the target tissue, and a second bleeder assembly is a "no-go bleeder", indicating to the user that if blood is dripping from an external, proximal opening, the suture mechanism is not positioned correctly (is over-inserted) with respect of the target tissue. In some embodiments, the go-bleeder assembly extends from an opening in the bridging portion at the distal region of suturing device, via a corresponding bleeder tube, extending from the distal port opening proximally along the shaft towards the proximal opening (outlet) located in the handle of the suturing device. In some embodiments, the "no-go" bleeder assembly extends from a distal opening in the PPM conduit in the shaft of the suturing device, and that conduit may optionally also serve as the no-go bleeder conduit, extending from the distal bleeder opening to the proximal opening, that may be further connected (via a manifold) to external bleeder tubes.

Reference is now made to **Figs. 11A-F,** illustrating different portions of bleeder assemblies, according to some embodiments. Shown in **Fig. 11A** are external bleeder tubes 602A-B, extending from handle 600 (having a part of the housing (a cover) removed for illustration purposes), wherein the tubes are connected/routed via manifold 604, from the internal bleeder tubes (configured to allow blood passage from the respective distal openings towards the proximal openings of the tubes), as shown in following figures. Further shown are drip stoppers 606A-B, configured to allow control of the blood dripping via the external tubes, as further detailed herein. Also shown is connecting element 556, PPM tube 560, and shaft 610. **Fig. 11B** shows transparent view of manifold 604 showing the internal openings 608A-B of external tubes 602A-B, each connected to a respective internal bleeder tube conveying blood from a distal opening (in the bridging portion or in the PPM conduit), to allow fluid communication therebetween. Further shown for illustration purposes is connecting element 556 (that may at least be partially housed within a tube/conduit, to prevent buckling thereof, while retracting the pocket (to a concealed position)), PPM tube 560, a tube 620 for suture thread routing (as further detailed below), and shaft 610. **Fig. 11C** shows an enlarged perspective view of bleeders manifold 604. As shown in Fig. 11C, manifold 604 is connected at a distal end thereof to shaft 610 and extending proximally via the manifold are connecting element 556 and PPM tube 560. Within the manifold (as illustrated in Fig. 11B), the internal bleeder tubes (i.e., "go bleeder" tube having a distal opening in the bridging portion and the "no-go" bleeder tube which is also conduit (tube) of the PPM) are connected/routed to external bleeder tubes 602A-B. At the end of each of the external tube, a dedicated drip stopper (i.e., drip stoppers 606A-B) are located. The drip stoppers are configured to prevent cross flow between bleeders during the operation of the device (in particular, during rotation thereof) and are further configured to prevent or hinder bleeders flow from reaching other portions of the handle. In some embodiments, the drip stoppers are bidirectional. Shown in **Figs. 11D-E** are close-up perspective views of exemplary drip stoppers 606A-B, As shown, drip stoppers 606A and 606B allow controlling blood flow, and prevent dripped blood from reaching operational areas, thereby hindering or otherwise affecting the suturing procedure, in particular while rotating the handle during the procedure, or holding the device (typically at an exemplary angle α of about 45 degrees). Reference is made to **Fig. 11F****,** which schematically shows distal openings of internal bleeder tubes, according to some embodiments. As shown in Fig. 11F, a first internal bleeder tube 630, includes a distal bleeder opening (outlet, port) 632, which is located in the bridging portion 640 of the suturing device. In some embodiments, the pocket is situated within an insert (shown, for example, in Fig. 19C), which includes, inter alia, a connecting conduit between the bleeder tube and the inlet port of the "GO" bleeder. In some embodiments, the proximal end of this insert is filled with flexible material, to block entry of blood from the pocket openings to the shaft. The bleeder opening and corresponding tube function as "go bleeder", indicating that if blood is flowing therethrough, the suturing device is correctly positioned in the tissue, for example, within a blood vessel. A second internal bleeder tube 634, which also functions as the PPM conduit (shuttle assembly 210 is shown), has a corresponding distal opening (outlet) 636, allowing blood flow therefrom, if the suturing device is not properly located (for example if it is over inserted into the tissue, more particularly, into a space/volume of the tissue such as, for example, a blood vessel). Thus, the second tube (PPM conduit) and opening function as a "no-go" bleeder, providing a visual indication that the device should be repositioned before the user attempts to perform a suturing operation (or the next stage thereof).

### Suture Storage Arrangement

According to some embodiments, the handle disclosed herein may further include an internal tube/channel/conduit for routing a suture thread (suture filament) to the distal end of the handle from a proximal region thereof. When operating the suturing device, each successive stitch requires dispensing of additional length of suture thread. As an alternative to various reels or other dispensing devices, it has been found particularly effective and reliable to provide the required length of suture filament preloaded into a thread tube, and to accommodate the thread tube at least partially within an internal volume of the device handle. The rounded flange ("torus") 16 described above with reference to Figs 4A-4C has been found to provide a convenient internal volume for housing a length of such a thread tube coiled around the axis of the device, ready to dispense suture thread via the suture thread tube, as the stitching process progresses. Reference is now made to **Figs. 12A-12C****,** which illustrate routing of suture thread tube through the handle, according to some embodiments. Shown in **Fig. 12A** is a partially cut-away perspective view of handle 22, showing the proximal, flat torus element 16. Further shown is the hollow chamber 704 of the flat torus element, which provide space for a suture thread tube (in particular, the coiled, proximal section thereof). Further shown is portion of suture thread tube 706, extending along the handle (partially hidden due to passing through a channel formed in the molded plastic. Shown in **Fig. 12B** is a cut-away view of a handle 22, illustrating flat torus element 16, having hollow chamber space 704, which can accommodate and route the coiled section 708 of suture thread tube 706. **Fig. 12B** shows the flat torus element 16 at the proximal region of the handle, illustrating the suture thread tube routing at region 708. **Fig. 12C** shows a top view of a cross section of flat torus element 16, demonstrating the suture thread tube 708 routing, along the hollow chamber of the flat torus element 16. Collectively, Figs. 12B and 12C show that the device handle cover (shell) includes: a central section having a longitudinal embedded conduit 705 to accommodate a portion of a suture thread tube; a proximal end section having a hollow flat torus shape with a peripheral channel like conduit at a perimeter thereof. The peripheral channel is capable of accommodating at least one coiled suture thread tube portion; and intermediate section having a 3-dimensional progressive helical-to-spiral curvature, channel like, conduit. The intermediate section connects the first (central) section with the second (proximal) section to create a continuous conduit for accommodating the suture thread tube while maintaining above a given minimum radius of curvature, thereby avoiding kinking of the tube and minimizing friction that might oppose drawing out of the suture as needed.

### Safety Catch Mechanism

According to some embodiments, as mentioned above herein, the handle proximal region may include a safety catch element and mechanism, configured to prevent unintentional activation of the device (in particular, by pressing the activating (suture) button), and moreover, may be used to prevent activation of the operating button (i.e., prevent it from being pressed), as long as the pocket mechanism has not been deployed. Such safety catch mechanism increases safety when utilizing the suturing device, by preventing unintended or untimely operation thereof. According to some embodiments, the safety catch element may be positioned/located such that it can physically interfere/prevent the activating (suture) button from being pressed, and only once removed/released, can the operating button function (i.e., capable of activating the suturing mechanism). In some embodiments, in order to increase safety and accuracy, the safety catch element may be locked in place and can only be removed/released after the pocket has been deployed (via activation of the pocket deployment mechanism).

Reference is now made to **Figs. 13A-D,** showing a safety catch mechanism, according to some embodiments. **Fig. 13A** shows safety catch 20 having a pull-tab 760 attached to a blocking element 762 which is configured to fit to/around a cylindrical portion 754 of a guide stem for operating button 14, to physically prevent the button from being pressed/moved. Clearly, depending on the particular structure employed for operating button 14, blocking element 762 may alternatively clip directly to a stem of the button, or may be adapted to any other form of force input which is used. Safety catch 20 is further engaged with flat torus element (hollow flange) 18, via a corresponding internal safety engagement (snap) mechanism, as detailed below herein. **Fig. 13B** shows safety catch 20 after it has been disengaged from the handle. Further shown in Fig. 13B is engagement (snap) element 764 of the safety catch, which is configured to associate via corresponding opening 766 in flat torus 18, with the internal safety engagement mechanism.

Reference is now made to **Fig. 13C****,** which shows a partial cross section of a flat torus element 18, showing portions of internal safety engagement mechanism, according to some embodiments. As shown in Fig. 13C, safety catch 20 is engaged with the handle. In addition, engagement (snap) element 764 with a corresponding edge 776 provided by hollow flange 18. Further shown is internal safety release mechanism 770, which includes at least a lever 774 which is associated with a push rod (not shown). The push rod is preferably associated/connected at a distal region thereof to the pocket deployment mechanism 400 or 550, described above, and for example, to the cam follower thereof. The push rod may be made of any suitable material, having sufficient rigidity to allow physically pushing the lever. As a result, only once the pocket deployment mechanism has been activated (for example, as detailed above, by turning an external knob and consequent movement of internal cam follower), the movement thereof induces upwards movement of the push rod which, in turn, induces upwards movement of pivotal lever 774 (for example by pushing the lever upwards), to thereby release snap element 764. In some embodiments, the rod may include a proximal bend to improve surface area interaction with the lever and is configured to push the lever upwards (proximally), consequently releasing the snap of the safety catch from the corresponding engagement region in the handle cover. In some embodiments, the lever has an inclined lower (distal) surface, which collides with the rod, and further causes it to move (rotate) sideways, immediately after the catch has been released, resulting in a proximal lever lock. Thus, only once the lever changes position (for example, by being pushed upwards, as detailed above), can the safety catch be released and removed from its location. In some embodiments, the lever is a pivotal lever, which can be pushed up/down to transition between engagement and release positions with respect of the corresponding snap element of the safety catch. In some embodiments, when the lever is pivoted upwards, in addition to releasing the engagement element of the safety catch from the stopper, is can also induce pushing of the safety catch outwards from its position. Such outward movement can indicate a user that the safety catch is ready to be moved.

### Needle and Holder Configurations

According to some embodiments, the shuttle (needle) disclosed herein has several regions, each configured to facilitate its operation and/or interaction with various components of the suturing mechanism during the suturing process. In some embodiments, the shuttle typically includes a distal end configured for tissue penetration (thereby defining it as a "needle"), a central (medial) section typically configured as an interface for suture thread engagement and pocket engagement interface, and a proximal end, typically configured as interface to the needle transmitter module (in particular, the PPM tube and PPM ejector). Reference is now made to **Fig. 14A****,** showing an exemplary shuttle, according to some embodiments. As shown in **Fig. 14A****,** shuttle 800, includes at a distal end 802 thereof a pointed or otherwise sharp tip 804, for piercing/puncturing a tissue. A middle (central) section 806 thereof, includes suture interface, which includes at least an aperture 809 for engaging/receiving a suture thread, and side recesses for accommodating the suture thread, preferably on both sides of the shuttle. The recesses may be only in the middle section of the shuttle, but optionally also in other sections of the shuttle. In some embodiments, the thread aperture may have a slotted shape, a circular shape, a constant diameter, or a tapered shape. As further shown in Fig. 14A, proximally to tip 804 and distally to suture interface 809, a pocket interface slot 810 is present. Pocket interface 810 may be in the form of a slot, a circular slot, a partially circular slot, in the body of the shuttle, and is configured to releasably engage a corresponding engagement element (for example, a snap ring) of the pocket. In some embodiments, the pocket interface may have a beveled profile at the lower (distal) portion thereof, along the entire surface of the slot, or along at least a portion thereof. As further detailed below, the pocket interface (for example in the form of a circular slot, beveled at the distal region thereof), allows receiving the corresponding engagement element of the pocket (for example, in the form of a flexible ring), to facilitate the holding of the needle by the pocket by limited axial holding forces (e.g., snap forces). As further shown in Fig. 14A, at a proximal region 812 thereof, the shuttle includes a proximal end 814 configured to interface with the needle transmitter module (i.e., the PPM tube and the PPM ejector). The proximal end 814 of the needle may be in the form of a truncated arrow, having an external diameter which is typically larger that the internal diameter of at least an end portion of the PPM tube. The proximal end 814 may include two graded portions 815A-B, wherein the distal portion 815A has a slightly larger diameter than the proximal, chamfered portion 815B. Furthermore, the proximal end may include a hole or indent, for example, an axial hole 816, that may be preferably chamfered, to thereby allow improved interaction with the needle ejector element of the needle transmitter module, as further detailed below.

As detailed above herein, the shuttle (needle) transmitter module (PPM) includes a shuttle (needle) holder (for example, in the form of a tube) configured for releasably holding the shuttle (in particular, by engaging a proximal region thereof), and a shuttle (needle) releaser/ejector element (for example, in the form of a rod), configured to interface with the proximal end of the shuttle, for example, by centrally aligning itself within a hole at the proximal end of the shuttle, and release said shuttle from the holder (tube). The shuttle holder may be made of any suitable material and may be rigid, semi-rigid or flexible. In some embodiments, the tube may be made of super elastic alloy, (such as, for example, Nitinol), plastic, metal, and the like. In some embodiments, the ejector element is configured to move in a reciprocating manner (in a proximal-distal direction), internally within the tube, to be able to engage a proximal region of the shuttle, most preferably under control of a mechanism such as was described above with reference to FIGS. 1A-7G. According to some embodiments, the shuttle transmitter may have any desirable shape to conform to the shape and/or size of the corresponding engagement proximal region/surface of the shuttle, to facilitate a secured, reversible engagement (holding) and releasing of the shuttle, as required, in accordance with the suturing steps. In some embodiments, the holding of the shuttle by the shuttle transmitter may be at sufficient force so as to prevent unintended or un-timely release thereof, but one which can still allow the ejector to release the shuttle from the transmitter module.

Thus, in accordance with some embodiments, the shuttle-shuttle transmitter module interface may be adjusted to ensure optimal interaction between the needle ejector and the shuttle. More particularly, such optimization can facilitate ejector engagement with the shuttle even under various degrees of off-center position of the ejector. To this aim, the proximal end of the shuttle may be sized so as to fit as closely as possible within the shuttle holder and may further include an engagement opening/hole/indentation configured to allow maximal surface area for interaction with the distal end of the ejector element.

Reference is now made to **Figs. 14B and 14C****,** showing views of shuttle-shuttle transmitter module interface, according to some embodiments. Shown in **Fig. 14B** is shuttle 800, with proximal end 814 thereof configured to interface with the distal region 852 of the transmitter module, in particular, with shuttle holder (PPM tube) 850, such that the proximal end of the shuttle can fit into the internal cavity of shuttle holder 850. As shown in Fig. 14B, the diameter (D1) of opening (hole) 816 at the proximal end 814 of the shuttle is substantially similar/identical to the internal diameter (D2) 858 of holder 850. Accordingly, advantageously, the chamfered regions in the distal end of the tube and the proximal end of the shuttle, allows the engagement therebetween, even when D1 is equal to or larger than D2. Thus, in some embodiments, having a chamfered region at the distal end of the tube allows interacting/holding a shuttle proximal end, even if said proximal end is not chamfered, or if the diameter of said proximal end hole (configured to associate/engage with the ejector of the transmitter module) is substantially similar to or larger than the internal diameter of the tube. Reference is now made to **Fig. 14C****,** which shows a cross section of a shuttle-shuttle transmitter module interface, while being associated with a shuttle receiver module 870. As shown in Fig. 14C, shuttle 800 is interfaced at a proximal end 814 thereof with shuttle transmitter module 870 (including shuttle holder (PPM tube) 850 and ejector 860), while also being associated (held by) with shuttle receiver module 870. The interface between the shuttle and the receiver module includes a snap association between the circular slot 867 having a beveled profile at the lower distal section of the shuttle and the corresponding flexible/elastic snap ring 865 in the receiver module, as described in detail herein below. As shown in Fig. 14C, the distal end 864 of ejector 860 (which may be pointed or otherwise having a penetrating shape) is capable of interfacing with chamfered opening 816 at the proximal end of shuttle 800. The size and/or shape of opening 816 can thus facilitate the engagement with the distal end (tip) of the ejector, under various conditions (for example, various degrees of off-center position of the ejector), thereby enhancing the ejection efficiency of the shuttle from the shuttle transmitter module. Thus, as shown in Fig. 14C, the shuttle is configured to be reversibly engaged both with the transmitter module and with the receiver module, via corresponding engagement elements of each of the modules.

By way of introduction to Figs. 15A-15D, various implementations of suturing devices according to the present invention impose corresponding requirements on the relative forces that should be required for engaging and releasing the shuttle. For example, in certain implementations, it is desirable for the force required for engaging and gripping the shuttle to be relatively small, and for the force required to extract the shuttle from the holder to be greater, so that the holder can reliably extract the shuttle from the shuttle receiver. At the same time, in certain implementations, the dimensions of the needle and the holder may be sub-millimeter in diameter, and in some cases less than half a millimeter in diameter. Presented here is a particularly advantageous form of engagement which has been effective for a wide range of dimensions, and in particular, has been found to be well-suited to sub-millimeter diameter applications.

As before, needle 800 is shows as having a pointed distal tip 804, an intermediate portion 806 configured to receive a suture and a proximal engagement portion 812. The proximal engagement portion 812 has a first section 813 adjacent to the intermediate portion and a second portion 814 proximal to the first portion. First portion 813 has a circumscribing cylinder of diameter D1 and length L1, and second portion 814 has a circumscribing cylinder of diameter D2 greater than D1 and a length L2. In the non-limiting example illustrated here, portions 813 and 814 are essentially cylindrical, such that a "circumscribing cylinder" corresponds to the outer surfaces of those portions (and is therefore not separately illustrated). However, also encompassed by these definitions are cases where these portions (particularly second portion 814) have a shape that departs from cylindrical, such as a cylinder modified by one or more flat chamfer surfaces or a hexagonal or other polygonal prism shape. In such cases, the "circumscribing cylinder" is the smallest virtual cylindrical construct which fully encloses the corresponding portion of the needle.

The corresponding holder design according to this aspect of the present invention is implemented as a tube 880, formed from super elastic material, the tube having a tip segment 882 of length no greater than L1 having an internal diameter matching diameter D1, and a second segment 884 having a length greater than L2 and an internal diameter matching diameter D2. The term "matching" in this context refers to a diameter of the tube segment which is essentially unstressed in order to accommodate the corresponding diameter of the engagement portion 812. This may correspond to what is referred to in engineering terms as a "tight fit", where the dimensions are substantially equal, according to the normal engineering tolerances for a tight fit, or a "slip fit", where the tube dimension is slightly greater than the corresponding portion of the engagement portion. The dimensions and tolerances to achieve such fitting are well-known in the engineering field. In all cases, the fit is preferably chosen to be sufficiently close to ensure stable axial alignment of the needle with the tube during operation.

As a result of this structure, when tube 880 is forced against the proximal end of needle 800, second portion 814 passes through the tip segment 882 of the tube causing elastic deformation of the tip segment, and when second portion 814 is fully inserted within second segment 884, tube 880 is substantially undeformed. Since the fully-inserted state of the needle corresponds to an undeformed state of tube 880, ejecting the needle from the holder encounters the resistance of deforming tip segment 882 from an undeformed state to a deformed state, which is fundamentally a similar transition to insertion of the needle, and therefore tends to enhance the resistance to extraction of the needle from the holder.

Additionally, according to certain particularly preferred implementations, a shape of the proximal engagement portion 812 of the needle and a design of the tube 880 are such that a force required to extract the needle from the holder when fully inserted is greater than a force required to insert the needle into the holder. This is typically achieved by suitable choice of the shape and angle of external surfaces at the transition between needle portions 812 and 814, and the shape of a conical chamfer surface at the proximal end of the needle, as well as design of the shape of the transition between the tip segment 882 and the second segment 884 of tube 880, as will be clear to one ordinarily skilled in the art.

In the case of Figs. 15A and 15B, the part of the tube 880 proximal to the second segment has an internal diameter the same as the tip segment of the tube. In this case, the enlarged second segment 884 can conveniently be formed by insertion of a suitably-shaped mandrel followed by heat treatment to fix the super-elastic shape memory of the tube material.

In the case of Figs. 15C and 15D, the proximal part of tube 880 continues proximally with an internal diameter equal to the internal diameter of second segment 884. In this case, the manufacturing technique starts with a tube of the larger dimension, and the narrower tip segment 882 is formed by use of an external template, with or without an internal mandrel, followed by heat treatment, all as is well-known in the art.

As before, the device also includes an ejector element 860 deployed within tube 880 and displaceable along the tube so as to eject the needle from the holder, most preferably under control of a mechanism such as was described above with reference to FIGS. 1A-7G.

Reference is made to **Figs. 16A-H,** showing alternative exemplary configurations of distal region of PPM tubes for interfacing with a shuttle, according to further embodiments. Shown in **Fig. 16A** is a distal end of PPM tube 900, having two openings (902A-B) on the tube. The openings may be positioned opposite thereto and may be identical or different with respect of size and/or form. In some embodiments, any number of openings may be present, for example, 1-4 openings. The openings, which may have any desired shape or size, are formed to fit a proximal region of a shuttle, to allow a snap action with the proximal end of the shuttle, to facilitate an engagement therebetween, to thereby enhance holding forces therebetween. This is of particular importance when pulling the shuttle from the receiver pocket. In some embodiments, as shown in Fig. 16A, the openings may have substantially elongated rectangular form, and may be positioned at a designated distance from the distal tip of the tube, so as to fit and engage a corresponding proximal portion 814 of the shuttle, having a truncated arrow shape, with a larger diameter. Reference is made to **Fig. 16B****,** which illustrates a shuttle 800 engaged with the distal region of PPM tube 900. In some embodiments, the most proximal end of the tube may be chamfered, and the inner diameter of the tube may be smaller than the diameter of the proximal end portion 814 of the shuttle, thereby enhancing the snap engagement between the tube (in particular, the openings thereof) and the shuttle. In some embodiments, the entire proximal region 814 of the shuttle (including sections 815A-B) is snapped/engaged with the openings of the tube.

Shown in **Fig. 16C****,** is an exemplary distal end of PPM tube 910, having partial openings 912A-B, on the external surface of the tube. The openings may be tooth-shaped and may be positioned opposite each other. The openings and may be identical, similar or different with respect of size and/or shape. In some embodiments any number of openings may be present, for example, 1-6. In some embodiments, the openings are sized to fit the proximal region of a shuttle, to allow a partial snap action therewith, to facilitate a reversible engagement therebetween, by allowing radial flexibility of the distal region of the tube, for accepting the shuttle proximal end. **Fig. 16D** illustrates a shuttle 800 engaged with the distal region of PPM tube 910, whereby the openings in the wall of the tube allows slight radial openings thereof, to allow engagement with the proximal region 814 of the shuttle. In such arrangement, enhanced axial stability of the shuttle may be facilitated, with less wear of the proximal end shape of the shuttle (said wear caused by repeated cycles of holding/engaging between the tube and the shuttle). **Fig. 16E** illustrates a shuttle 800 engaged with a distal region of PPM tube 920, having at least one opening 922A on the tube, whereby the opening is located at a distance from the distal end of the tube, and is optionally square, to allow engagement with the proximal region of the shuttle.

Shown in **Fig. 16F****,** is an exemplary distal end of PPM tube 930, having slotted openings (exemplary slotted openings 932A-E are shown), along the circumference of the surface of the tube. Each of the openings may be in the form of narrow rectangular slot and the openings may be substantially homogenously distributed along the circumference of the tube. The slots may be identical, similar or different with respect of size and/or shape. In some embodiments, the slots are sized and positioned so as to fit the proximal region of a shuttle, to allow a snap action therewith, to facilitate a reversible engagement therebetween. **Fig. 16G****,** illustrates a shuttle 800 engaged with the distal region of PPM tube 930, whereby the slots in the wall of the tube collectively form a snap engagement element with the proximal region of the shuttle. In such arrangement, enhanced axial stability of the shuttle may be facilitated and less degradation of the external diameter of the proximal end shape thereof may be required, to allow sufficient interaction with the shuttle holder. **Fig. 16H** illustrate additional exemplary openings 942A-B, on the distal region of a PPM tube end, according to some embodiments.

### Pocket Needle-Retention Features

As detailed above herein, the receiver module (also referred to as pocket module) is configured to passively accept and securely hold the shuttle, once ejected from the shuttle transmitter, and to passively release the shuttle to the shuttle transmitter, in accordance with the suturing steps. Reference is now made to Figs. **17A-C,** which illustrate views of a receiver module, according to some embodiments. **Figs. 17A-B** show full and partially cut-away isometric views of pocket 1000, associated with/attached to the distal end of connecting element 1002 (corresponding to element 556, configured to allow retraction/deployment of the pocket, by the control of the pocket deployment mechanism, located in handle of the suturing device, as detailed above). As better seen in the cross-sectional view of FIG. 17D, connecting element 1002 may have a flattened (or ball-shaped, not shown) distal end 1008, allowing securing/anchoring of the connecting element to the body of the pocket, by any suitable means, such as, pins, screws, rings, and the like. In some embodiments, a ring or sleeve 1009 may be threaded as a bead onto the connecting element to function as a strain releaser / distributor at the anchoring interface of the connecting element located in a stepped hole in the pocket.

Further shown is shuttle receiving portion 1004, which is shaped to accommodate a corresponding shuttle 800. Further shown is engagement element 1006, configured to interact with corresponding grooves 810 of a shuttle, to facilitate the holding of the shuttle within the receiving portion 1004, for example, by axial holding forces of limited magnitude (i.e., snap forces), as further illustrated below. Engagement element 1006 includes a front region, in the shape of a ring, or other circular form, corresponding to the shape of the corresponding grooves in a shuttle to be engaged, and a back region configured to allow securing/engaging/anchoring the engagement element to the body of the pocket, using any suitable means, such as, pins, screws, rings, and the like. In some embodiments, the engagement element is fitted to a corresponding slot/opening 1014. **Fig. 17C** shows a perspective view of an exemplary engagement element 1006. As shown in Fig. 17C, engagement element 1006 (which is configured to fit into slot 1014), includes a shuttle engagement portion 1012A, which is configured to associate with a shuttle (in particular, with corresponding engagement grooves 810 of a shuttle, as shown, for example, in Fig. 14A), and a pocket engagement portion 1012B, configured to allow association with the pocket body. In the example shown in Fig. 17B, the different portions of engagement element 1006 may be integrally formed as parts of a substantially flat unitary element. In some embodiments, the engagement element is flexible and may be made of suitable materials, such as, for example, but not limited to: plastic, nylon, silicone, rubber, or preferably from super elastic alloy, such as, for example, Nitinol. In some embodiments, the size and/or shape of the engagement element may be adjusted/predetermined in accordance with the size, form and/or shape of the shuttle to be engaged. In some embodiments, the shuttle engagement portion 1012A may be in the form of a ring and may optionally include an opening/slot 1015 (i.e., incomplete ring form), to allow flexibility in the holding (snap) and release of the shuttle. In some embodiments, Portion 1012A is connected to portion 1012B via a beam like or otherwise flexible element 1013, to facilitate the centering and expansion of the ring during needle engagement. The tolerances of pocket engagement portion 1012B and/or the flexibility of the flexible connecting element 1013 interconnecting the pocket engagement portion with the shuttle engagement portion 1012A are preferably such that the shuttle engagement portion is effectively "floating" in the sense that it can move freely within a sufficient range of positions within slot 1014 so as to self-align with shuttle 800 as the shuttle is inserted into the shuttle receiving portion. This freedom of motion should be sufficiently limited to ensure that the leading tip of the shuttle will successfully locate itself within the opening of the shuttle engagement portion when inserted.

Reference is made to **Fig. 17D****,** showing a cross section of a pocket assembly, according to some embodiments. Shown in **Fig. 17D** is pocket 1000, while being associated with shuttle 800, which is housed in receiving portion 1004 of the pocket. As shown in Fig. 17D, engagement element 1006 (in particular, the ring portion thereof), is associated with the body of shuttle 800, in particular, with pocket interface slot 810 of the shuttle. The slot/groove 810 along the circumference of the medial region/lower distal section of the shuttle fits into and held by the ring portion of engagement element 1006. Such limited axial holding forces (snap holding) by the ring, secures the needle to the pocket, when being held by the pocket. As further shown in Fig. 17D, the shuttle receiving portion 1004, includes an upper (proximal) section 1017A, a medial section 1017B and a lower (distal) section 1017C. The proximal region 1017A has a relatively large diameter, allowing entry/acceptance of the shuttle, even if not centered, as well as providing space for the suture thread associated with the shuttle (on the sides thereof), and allowing the tube distal end to at least partially radially expand, to allow releasing of the shuttle therefrom. The medial region 1017B allows axial stabilization of the shuttle within the receiver, and includes the slot 1014, for accepting the engagement element 1006. The distal region 1017C, includes a gradually or otherwise-decreased diameter bore, for halting/stopping the axial movement of the shuttle.

Reference is made to **Figs. 18A-D**, which show further exemplary preferred embodiments of receiver modules having a shuttle engagement element. As in the previous embodiment, the receiver body (pocket) has a needle-receiving bore extending parallel to a bore axis for receiving the needle and a retaining-element slot extending from a side of the receiver body and intersecting the needle-receiving bore. A resilient snap retainer is deployed in the retaining-element slot so that the resilient snap retainer is aligned within the needle-receiving bore for resiliently retaining the needle.

In the case of Figs. 18A-18B, receiver module (pocket) 1050 has a shuttle receiving portion (needle-receiving bore) 1054, which is shaped to accommodate a corresponding shuttle. Additionally shown is engagement element 1056, wherein an inner ring (resilient snap retainer) 1060 thereof, is configured to interact with corresponding grooves of a shuttle, to facilitate the holding of the shuttle within the receiving portion 1054. As shown in Fig. 18A, an inner ring 1060, may be slotted and is located/positioned within a housing/body portion 1062 which preferably traps the resilient snap retainer in the required range of positions while leaving sufficient clearance for it to "float" to self-align with the shuttle on insertion into the needle-receiving bore. The housing is configured to fit into a corresponding front opening, (slot) in the body of pocket 1050, and may be secured thereto using securing geometries 1064A-B. Securing geometries 1064A-B, may be in the form of flexible extensions (resilient locking tabs or "wings"), slightly protruding upwards, and are configured to snap/lock with a corresponding opening 1067 in pocket 1050.

The implementation of Figs. 18C-18D is similar to that of Figs. 18A-18B, but employs an anchoring configuration similar to that of Figs. 17A-17D. Specifically, in this case, the receiver body further includes a locking element channel intersecting with the retaining-element slot, and the resilient snap retainer is interconnected with an anchoring configuration having an aperture aligned with the locking element channel. A locking element (pin) 1110 is deployed in the locking element channel so as to engage the aperture, thereby anchoring the resilient snap retainer in alignment with the needle-receiving bore.

Thus, receiver module (pocket) 1100 has a shuttle receiving portion (cavity) 1104, which is shaped to accommodate a corresponding shuttle. Additionally shown is engagement element 1106, which includes an inner ring 1112, which may be slotted, and located within a housing/body 1114. The housing 1114 further includes a slot 1117, beveled at the rear end thereof (1119), separating the end portion of the housing to two end sections 1115A-B. The engagement module is configured to fit into a corresponding (slot) in the body of pocket 1110, and may be secured thereto using pin 1110 attached to the pocket. The engagement element (in particular, housing thereof) is configured to snap around the pin, by virtue of chamfered slot 1117, sliding over the pin 1110, while spreading apart the open end sections 1115A-B of the housing. In some embodiments, the engagement element (housing and/or ring) may be flexible or semi flexible, and may be made of any suitable material, such as, but not limited to: plastic, rubber, silicon, super elastic alloy (such as Nitinol), and the like, or any combination thereof.

In each of the non-limiting examples of Figs. 17A-18D, the resilient snap retainer is a snap ring which extends around the entire periphery of the shuttle. This is particularly valuable for shuttle designs such as the needle 800 illustrated in Fig. 14A, where peripheral groove 810 does not extend around the entire needle. In this case, the use of a ring encircling the shuttle ensures snap retention of the shuttle independent of the rotational orientation in which it arrives to the pocket. In other implementations, for example, where the peripheral groove surrounds the shuttle, other snap retainers, such as a leaf-spring wire on one or two sides of the needle-receiving bore, may be used.

In certain particularly preferred implementations, the shapes and mechanical design of the peripheral groove and the resilient snap retainer are such that a force required to release the needle from the needle receiver is greater than a force required to engage the needle in the needle receiver.

### Shuttle Transmitter Self-Alignment in Shuttle Receiver

A further particularly preferred feature of certain embodiments of a suturing device according to an aspect of the present invention is illustrated in Figs. 19A-19D and 20. According to this aspect of the present invention, the geometry of the shuttle receiver 1000, the shuttle 800 and the shuttle transmitter 210 are such that, when the shuttle is seated in the bore 1004 of the shuttle receiver, the shuttle transmitter will self-align coaxially with the shuttle to ensure correct operation of the shuttle holder for engaging the shuttle and withdrawing it from the receiver bore, so long as the initial approach of the shuttle transmitter axis lies anywhere within the shuttle receiver bore. This process is illustrated in Figs. 19A-19D, where Fig. 19D illustrates an initial approach of the shuttle transmitter which lies significantly off-axis relative to the receiver bore, but just within the outer perimeter thereof. Fig. 19B illustrates how, prior to reaching the shuttle, the shuttle transmitter is guided by sliding of its surfaces over peripheral surfaces of the bore until the tip of the shuttle transmitter lies within the rim of an axial aperture 816 of the shuttle, so that further motion of the shuttle transmitter self-aligns with that aperture, as seen in Fig. 19C. The shuttle transmitter is then correctly aligned for engagement of the shuttle holder, as illustrated in Fig. 19D.

The primary geometrical requirement to ensure this self-alignment process is illustrated schematically in Fig. 20, which is an enlarged and annotated partial view of Fig. 19A. The proximal engagement portion of the shuttle presents an axial aperture surrounded by a rim having a radius R2 from a central axis of the shuttle. The bore of the shuttle receiver has an opening which has a radius R1 and is located at an axial height H from the rim of the axial aperture of the shuttle when the shuttle is in the inserted position. Using this terminology, when the shuttle transmitter configuration assumes a penetrating configuration terminating in a penetrating point, this self-centering functionality can be provided by ensuring that the radius of the penetrating configuration gradually-increases such that, at an axial distance H from the penetrating point, the penetrating configuration has a radius R3, where R3 is greater than (R1 - R2).

### Dilator Swivel Configuration

As mentioned above, the suturing device may further optionally include a dilator, connected to a distal end of the shaft, via a dedicated connector. Such flexible dilator is deployed distally to the shaft of the suturing mechanism (the bridging portion of the device) and is interconnected therewith via a swivel connector. In some embodiments, the dedicated dilator connector is a swivel connector having a fixed angle.

Reference is now made to **Figs. 21A-E,** showing various views of a swivel connector and associated interfaces, according to some embodiments. **Fig. 21A(i)-(iii)** show three views of the swivel connector 1200, which connects/bridges the distal end of the suturing mechanism (shaft thereof) 1202 and the proximal end of dilator 1204, illustrating three different fixed-angle connectors to provide a user-selectable working angle on assembly of the device. As shown in Fig. 21A(i)-(iii), connector 1200 may include one or more openings/holes 1206 for securing the swivel connector to the suturing mechanism portion (in particular to an internal insert thereof, as detailed below). The securing of the swivel connector to the suturing mechanism portion/module may be facilitated by any suitable means, including, for example, screw, pins, welding (for example, by spot welding, laser welding, etc.), and the like. In some embodiments, the connector may be secured to a locking sleeve (lock ring) associated with the internal insert most distal end, having a shape of an axis/hinge, as detailed below. **Fig. 21B** shows a partial cross section view of Fig. 21A(i), in which connector 1200 and dilator 1204 are shown in cross section view. As can be seen in Fig. 21B, the distal end of shaft of the suturing mechanism 1202 includes an insert distal end 1208, which is configured to connect to (hold) connector 1200 and further allow swiveling of the connector thereabout. Insert 1208 is fixed to the distal end of the suturing mechanism (also referred to as mast end), by any suitable means. In some embodiments, the insert may be integrally formed with the shaft of the suturing mechanism. In some embodiments, no relative movement is formed between the insert and the shaft, as both these portions are stationary. As shown in Fig. 21B, insert 1208 has an elongated body having integrated slip bearings (collars) at the proximal end and at the distal end thereof (1210A-B, respectively). The middle section (having a recessed diameter) 1212 of the insert distal end is configured to associate with a locking sleeve (lock ring 1214). The lock ring is configured to tightly fit (snap on) the diametrically recessed middle-section of the insert distal end and to further interact with a corresponding opening 1206 (Figs. 21A(i)-(iii)) in connector 1200, to allow securing the connector to the insert distal end. In some embodiments, the connector may be further secured to the locking sleeve (lock ring), for example, by welding thereto (via, for example, via opening 1206).

Fig. 21C shows a cross section of a suturing mechanism distal end, connected to a dilator, via a swivel connector. Distal end 1202 of the suturing mechanism, includes receiver module 1250 (shown in retracted (concealed) position) also shown is connecting element 1252, connecting the receiver module to the proximal PDM), as well as shaft 1254 of the transmitter module. An elastomeric seal 1253 preferably prevents leakage of blood along the channel of connecting element 1252. Further shown is insert 1256 which is fixed to the shaft of the suturing mechanism and having a distal end (mast end) 1208, protruding therefrom. As detailed above, the insert distal end 1208 includes integrated slip bearings at the proximal and distal ends thereof, and a middle section having a recessed diameter, which is configured to associate with a locking sleeve 1214. Further shown is swivel connector 1200, which is connected to the distal end of insert 1208 (which serves as an axis for the rotation of connector 1200), and further connected, via a connecting element (shown as barbed connector 1220), to dilator 1204.

**Fig. 21D** shows a perspective view of the distal end of insert 1256, illustrating insert distal end 1208, and locking sleeve 1214 associated therewith. In some embodiments, the locking sleeve may include a slot 1216, to facilitate the assembly/placement of the sleeve over the insert distal end. In some embodiments, the locking sleeve may be snapped over the insert. In some embodiments, the locking sleeve can rotate about the insert. In some embodiments, the locking sleeve can increase the diameter of the middle, recessed section of the distal end of the insert. In some embodiments, the locking sleeve facilitates axial locking of the insert distal end with the swivel connector, by forming a tight fit between the locking sleeve and a corresponding opening/receiving portion of the swivel connector. **Fig. 21E** shows a cross section view of connector 1200 associated with insert distal end 1208. As shown, the connector can rotate over the 2 collar slide bearings 1210A-B, and the locking sleeve further provides a free rotatable interface. Referring back to **Fig. 21B****,** swivel connector 1200 further includes at a distal end thereof a connecting element 1220, configured to connect/attach/secure dilator 1204 thereto. In some embodiments, the distal connecting element 1220 may be integrally formed with connector 1200 or may be attached/associated therewith. In some embodiments, connecting element 1220 may have an elongated body, having one or more engagement elements, configured to enhance the interaction/association between the dilator and the distal connecting element. In some exemplary embodiments, as shown in Fig. 21B, distal connecting element 1220 may be a barbed connector, having at a distal region thereof, protruding structures 1222, (shown for example, in the form of miniature circular teeth). In some embodiments, the distal connecting element may fit into a corresponding opening 1224, located at the proximal region of the dilator body. In some embodiments, the connecting element and the dilator may be integrally formed. In some embodiments, the connecting element may be embedded with the dilator.

In some embodiments, the connector is a fixed angle connector, which may be at a selected angle in the range, for example, from 0 degrees deflection (straight) up to 90 degrees deflection (perpendicular), between the shaft of the suturing mechanism and the dilator. According to some embodiments, the angle may be predetermined and accordingly, a corresponding connector may be defined for various pre-shaped angles. In some embodiments, selection of an angle of the connector for use may be determined by a specific need such as a medical procedure, and the like. A set of typical preferred angles may include some or all of the angles illustrated in FIGS. 21A(i)-(iii), namely, 20 degrees, 30 degrees and 40 degrees, and/or additional angle options.

According to some embodiments, there is provided herein a method of suturing a material (such as, an in-vivo tissue) utilizing the suturing device as disclosed herein.

According to some embodiments, while the presented disclosure is describing a device with some of the components connected with fasteners such as screws and nuts, it should be understood that at any of these incidents these connections may be achieved by US welding, gluing, snaps and alike.

It is appreciated that certain features of the disclosure, which are, for clarity, described in the context of separate embodiments, may also be provided in combination in a single embodiment. Conversely, various features of the disclosure, which are, for brevity, described in the context of a single embodiment, may also be provided separately or in any suitable subcombination or as suitable in any other described embodiment of the disclosure. No feature described in the context of an embodiment is to be considered an essential feature of that embodiment, unless explicitly specified as such.

As used herein, the indefinite articles "a" and "an" mean "at least one" or "one or more" unless the context clearly dictates otherwise.

Although steps of methods according to some embodiments may be described in a specific sequence, methods of the disclosure may include some or all of the described steps carried out in a different order. A method of the disclosure may include a few of the steps described or all of the steps described. No particular step in a disclosed method is to be considered an essential step of that method, unless explicitly specified as such.

Although the disclosure is described in conjunction with specific embodiments thereof, it is evident that numerous alternatives, modifications and variations that are apparent to those skilled in the art may exist. Accordingly, the disclosure embraces all such alternatives, modifications and variations that fall within the scope of the appended claims. It is to be understood that the disclosure is not necessarily limited in its application to the details of construction and the arrangement of the components and/or methods set forth herein. Other embodiments may be practiced, and an embodiment may be carried out in various ways.

The phraseology and terminology employed herein are for descriptive purpose and should not be regarded as limiting. Citation or identification of any reference in this application shall not be construed as an admission that such reference is available as prior art to the disclosure. Section headings are used herein to ease understanding of the specification and should not be construed as necessarily limiting.

As used herein, the term "about" may be used to specify a value of a quantity or parameter (e.g., the length of an element) to within a continuous range of values in the neighborhood of (and including) a given (stated) value. According to some embodiments, "about" may specify the value of a parameter to be between 80 % and 120 % of the given value. According to some embodiments, "about" may specify the value of a parameter to be between 90 % and 110 % of the given value. According to some embodiments, "about" may specify the value of a parameter to be between 95 % and 105 % of the given value.

In the description and claims of the application, each of the words "comprise" "include" and "have", and forms thereof, are not necessarily limited to members in a list with which the words may be associated.

CLAUSES DESCRIBING INVENTION(S) PRESENTED HEREIN:
1. A mechanism for operating a medical device comprising:
   (a) a handle;
   (b) an effector assembly mounted relative to said handle so as to be displaceable along an axial direction relative to said handle, said effector assembly comprising:
      (i) a first effector,
      (ii) a second effector,
      (iii) a bistable mechanism including a bistable element, and
      (iv) a biasing arrangement including at least a first spring element and a second spring element, said biasing arrangement biasing each of said first and second effectors in a distal direction relative to said bistable element, said bistable element assuming a first axial state in which said second effector is biased towards a first relative axial position relative to said first effector and a second axial state in which said second effector is biased towards a second relative axial position relative to said first effector; and
   (c) a force input deployed to selectively apply an input force to said bistable mechanism,
   wherein a force applied by said force input in a first direction is effective sequentially:
   (i) to displace said effector assembly distally along said axial direction without changing a state of said bistable mechanism, and
   (ii) when at least part of said effector assembly encounters an obstacle to further distal displacement, to toggle said bistable element between said first axial state and said second axial state,
   and wherein said biasing arrangement is configured such that said bistable mechanism can be toggled from said first state to said second state without requiring said second effector to have reached said second relative axial position.
2. The mechanism of claim 1, wherein said first spring element acts between said first effector and said second effector, and wherein said second spring element acts between said second effector and said bistable element.
3. The mechanism of claim 1, wherein said first spring element acts between said first effector and said bistable element, and wherein said second spring element acts between said second effector and said bistable element.
4. The mechanism of claim 1, further comprising a retraction spring deployed to return said bistable mechanism and said first and second effectors along the axial direction in a proximal direction.
5. The mechanism of claim 1, wherein said first effector is a holder for holding a suture needle, and wherein said second effector is an ejector effective when displaced from said first relative axial position to said second axial relative position to eject the suture needle from said holder.
6. The mechanism of claim 5, wherein, in said second axial relative position, said ejector presents a penetrating tip.
7. The mechanism of claim 1, wherein at least one of said first and second springs is deployed with a preload force that defines a minimum force required to change a length of the at least one spring.
8. The mechanism of claim 1, wherein, in said first axial state of said bistable element, said first spring is deployed with a first preload force and said second spring is deployed with a second preload force, and wherein, in said second axial state of said bistable element, at least one of said first and second preload forces changes such that a ratio between said first preload force and said second preload force differs between said first axial state and said second axial state.
9. A suturing mechanism comprising:
   (a) a needle having a pointed distal tip, an intermediate portion configured to receive a suture and a proximal engagement portion, said proximal engagement portion comprising a first section adjacent to said intermediate portion and a second portion proximal to said first portion, said first portion having a circumscribing cylinder of diameter D1 and length L1, and said second portion having a circumscribing cylinder of diameter D2 greater than D1 and a length L2; and
   (b) a holder for releasably holding said needle, said holder comprising a tube formed from super elastic material, said tube having a tip segment of length no greater than L1 having an internal diameter matching diameter D1, and a second segment having a length greater than L2 and an internal diameter matching diameter D2,
   such that, when said tube is forced against a proximal end of said needle, said second portion passes through said tip segment of said tube causing elastic deformation of said tip segment, and when said second portion is fully inserted within said second segment, said tube is substantially undeformed.
10. The suturing mechanism of claim 9, wherein a shape of said proximal engagement portion of said needle and a design of said tube are such that a force required to extract said needle from said holder when fully inserted is greater than a force required to insert said needle into said holder.
11. The suturing mechanism of claim 9, wherein a part of said tube proximal to said second segment has an internal diameter the same as said tip segment of said tube.
12. The suturing mechanism of claim 9, wherein said tube continues proximally to said second segment with an internal diameter equal to the internal diameter of said second segment.
13. The suturing mechanism of claim 9, further comprising an ejector element deployed within said tube and displaceable along said tube so as to eject said needle from said holder.
14. A needle receiver for passively retaining a needle of a suturing device, the needle receiver comprising:
   (a) a receiver body having a needle-receiving bore extending parallel to a bore axis for receiving the needle and a retaining-element slot extending from a side of said receiver body and intersecting said needle-receiving bore; and
   (b) a resilient snap retainer deployed in said retaining-element slot so that said resilient snap retainer is aligned within said needle-receiving bore for resiliently retaining the needle.
15. The needle receiver of claim 14, wherein said receiver body further comprises a locking element channel intersecting with said retaining-element slot, and wherein said resilient snap retainer is interconnected with an anchoring configuration having an aperture aligned with said locking element channel, the needle receiver further comprising a locking element deployed in said locking element channel so as to engage said aperture, thereby anchoring said resilient snap retainer in alignment with said needle-receiving bore.
16. The needle receiver of claim 15, wherein said resilient snap retainer and said anchoring configuration are interconnected via a flexible connecting element so as to facilitate self-alignment of said resilient snap retainer with the needle inserted into said needle-receiving bore.
17. The needle receiver of claim 16, wherein said resilient snap retainer, said flexible connecting element and said anchoring configuration are integrally formed as a unitary flat element made of super elastic material.
18. The needle receiver of claim 14, wherein said resilient snap retainer is a snap ring.
19. The needle receiver of claim 14, wherein said needle-receiving bore has an internally-stepped bore defining a fully-inserted position of the needle.
20. The needle receiver of claim 14, further comprising a needle for introducing into the needle receiver, said needle having a peripheral groove for receiving said resilient snap retainer, and wherein said groove and said resilient snap retainer are configured such that a force required to release said needle from the needle receiver is greater than a force required to engage said needle in the needle receiver.
21. A suturing mechanism comprising:
   (a) a shuttle having an intermediate portion configured to receive a suture and a proximal engagement portion, said proximal engagement portion presenting an axial aperture surrounded by a rim having a radius R2 from a central axis of said shuttle;
   (b) a shuttle receiver having a bore for receiving and releasably holding said shuttle in an inserted position, said bore having an opening which has a radius R1 and is located at an axial height H from said rim of said axial aperture of said shuttle when in said inserted position; and
   (c) a shuttle transmitter configuration for engaging said shuttle within said bore, said shuttle transmitter assuming a penetrating configuration in which said shuttle transmitter terminates in a penetrating point, said penetrating configuration having a gradually-increasing radius such that, at an axial distance H from said penetrating point, said penetrating configuration has a radius R3, where R3 is greater than (R1 - R2).

## Claims

1. A suturing mechanism comprising:
(a) a shuttle (800) having an intermediate portion (806) configured to receive a suture and a proximal engagement portion (812), the proximal engagement portion (812) presenting an axial aperture (816) surrounded by a rim having a radius R2 from a central axis of the shuttle (800);
(b) a shuttle receiver (1000) having a bore (1004) for receiving and releasably holding the shuttle (800) in an inserted position, the bore (1004) having an opening which has a radius R1 and is located proximally at an axial height H from the rim of the axial aperture (816) of the shuttle (800) when in the inserted position; and
(c) a shuttle transmitter configuration (210) for engaging the shuttle (800) within the bore (1004), the shuttle transmitter (210) assuming a penetrating configuration in which the shuttle transmitter (210) terminates in a penetrating point, the penetrating configuration having a gradually-increasing radius such that, at an axial distance H from the penetrating point, the penetrating configuration has a radius R3, where R3 is greater than (R1 - R2).

2. The suturing mechanism of claim 1, wherein the shuttle transmitter (210) comprises a shuttle holder tube (850, 880) and an ejector rod (860) deployed within, and axially displaceable relative to, the shuttle holder tube (850, 880), the ejector rod (860) projecting from the shuttle holder tube (850, 880) in the penetrating configuration and providing the penetrating point.

3. The suturing mechanism of claim 2, wherein the bore (1004) has a proximal section (1017A) sized to allow a distal end of the shuttle holder tube (850, 880) to engage the proximal engagement portion (812) of the shuttle (800).

4. The suturing mechanism of claim 3, wherein the proximal section (1017A) of the bore (1004) is sized to allow at least part of the distal end of the shuttle holder tube (850, 880) to expand radially.

5. The suturing mechanism of any one of claims 2 to 4, wherein part of the gradually-increasing radius is provided by the ejector rod (860) and part of the gradually-increasing radius is provided by the shuttle holder tube (850, 880).

6. The suturing mechanism of any one of claims 2 to 4, wherein the proximal engagement portion (812) comprises a first section (813) adjacent to the intermediate portion (806) and a second section (814) proximal to the first section (813), the first section (813) having a circumscribing cylinder of diameter D1 and length L1, and the second section (814) having a circumscribing cylinder of diameter D2 greater than D1 and a length L2, and wherein the shuttle holder tube (880) is formed from super elastic material, the shuttle holder tube (880) having a tip segment (882) of length no greater than L1 having an internal diameter matching diameter D1, and a second segment (884) having a length greater than L2 and an internal diameter matching diameter D2, such that, when the shuttle holder tube (880) is forced against a proximal end of the shuttle (800), the second section (814) passes through the tip segment (882) of the shuttle holder tube (880) causing elastic deformation of the tip segment (882), and when the second section (814) is fully inserted within the second segment (884), the shuttle holder tube (880) is substantially undeformed.

7. The suturing mechanism of claim 6, wherein a shape of the proximal engagement portion (812) of the shuttle (800) and a design of the shuttle holder tube (880) are such that a force required to extract the shuttle (800) from the shuttle holder tube (880) when fully inserted is greater than a force required to insert the shuttle (800) into the shuttle holder tube (880).

8. The suturing mechanism of any one of claims 3-7, wherein the proximal section (1017A) of the bore (1004) is sized to accommodate a suture thread associated with the shuttle (800).

9. The suturing mechanism of any preceding claim, wherein the shuttle receiver (1000) is configured for passively retaining the shuttle (800) and comprises a receiver body in which the bore (1004) extends parallel to a bore axis, the receiver body having a retaining-element slot (1014) extending from a side of the receiver body and intersecting the bore (1004), and wherein the shuttle receiver (1000) further comprises a resilient snap retainer (1012A, 1060, 1112) deployed in the retaining-element slot (1014) so that the resilient snap retainer (1012A, 1060, 1112) is aligned within the bore (1004) for resiliently retaining the shuttle (800).

10. The suturing mechanism of claim 9, wherein the receiver body further comprises a locking element channel intersecting with the retaining-element slot (1014), and wherein the resilient snap retainer (1012A, 1112) is interconnected with an anchoring configuration (1012B, 1114) having an aperture aligned with the locking element channel, the shuttle receiver (1000) further comprising a locking element (1110) deployed in the locking element channel so as to engage the aperture, thereby anchoring the resilient snap retainer (1012A, 1112) in alignment with the bore (1004).

11. The suturing mechanism of claim 10, wherein the resilient snap retainer (1012A) and the anchoring configuration (1012B) are interconnected via a flexible connecting element (1013) so as to facilitate self-alignment of the resilient snap retainer (1012A) with the shuttle (800) inserted into the bore (1004).

12. The suturing mechanism of claim 11, wherein the resilient snap retainer (1012A), the flexible connecting element (1013) and the anchoring configuration (1012B) are integrally formed as a unitary flat element (1006) made of super elastic material.

13. The suturing mechanism of claim 9, wherein the resilient snap retainer (1012A, 1060, 1112) is a snap ring.

14. The suturing mechanism of any preceding claim, wherein the bore (1004) is an internally-stepped bore defining the inserted position of the shuttle (800).

15. The suturing mechanism of any preceding claim, wherein the shuttle (800) has a pointed distal tip (804).
